# EUROPÄISCHE PATENTSCHRIFT

(11) **EP 1 000 123 B1**
(45) Veröffentlichungstag und Bekanntmachung des Hinweises auf die Patenterteilung: **10.09.2008**
(21) Anmeldenummer: 98939609.8
(22) Anmeldetag: 03.07.1998
(51) Int. Cl.: C09B 62/00

(54) **VERWENDUNG VON REAKTIVFARBSTOFFEN ZUM FÄRBEN VON HAAREN**
USE OF REACTIVE DYES FOR DYEING HAIR
UTILISATION DE COLORANTS REACTIFS POUR LA TEINTURE DES CHEVEUX

(30) Priorität: 21.07.1997 DE 19731166
(43) Veröffentlichungstag der Anmeldung: 17.05.2000
(73) Patentinhaber: BASF SE, 67056 Ludwigshafen (DE)
(72) Erfinder: PRECHTL, Frank, D-68167 Mannheim (DE); PATSCH, Manfred, D-67157 Wachenheim (DE); HÖSSEL, Peter, D-67105 Schifferstadt (DE)
(86) Internationale Anmeldenummer: PCT/EP1998/004104
(87) Internationale Veröffentlichungsnummer: WO 1999/005222

(56) Entgegenhaltungen:
- EP-A- 0 181 585
- EP-A- 0 433 764
- EP-A- 0 437 006
- EP-A- 0 559 617
- EP-A- 0 733 680
- EP-A- 0 798 347
- DE-A- 2 154 942
- US-A- 4 066 638
- US-A- 4 102 641

## Beschreibung

Die vorliegende Erfindung betrifft die Verwendung von Reaktivfarbstoffen der Formel I 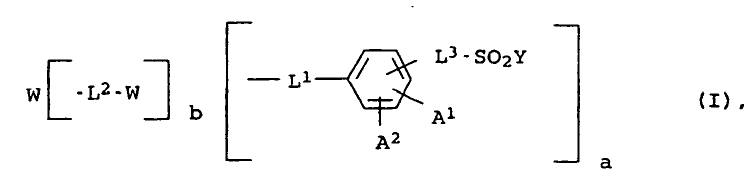 in der
- a: 1 oder 2,
- b: 0 oder 1,
- Y: Vinyl oder einen Rest der Formel C₂H₄Q, worin Q für eine unter alkalischen Reaktionsbedingungen abspaltbare Gruppe steht,
- L³: eine direkte Bindung oder ein Brückenglied der Formel CO-NH-M¹, worin M¹ für C₂-C₆-Alkylen steht, daß durch 1 oder 2 nicht benachbarte Sauerstoffatome, Imino- oder C₁-C₄-Alkyl-iminogruppen unterbrochen sein kann,
- A¹: Hydroxysulfonyl oder einen Rest der Formel SO₂Y
- A²: Wasserstoff, Hydroxysulfonyl, Methoxy, Chlor, Brom oder Carboxyl
- W: im Fall 1) den Rest einer Kupplungskomponente, eines Monoazofarbstoffs oder zusätzlich, wenn b = 0, eines Disazofarbstoffs, die weitere faser-reaktive Gruppen tragen können, oder
im Fall 2) den Rest eines Chromophors, der gegebenenfalls weitere faserreaktive Gruppen aufweist und der sich von einem gegebenenfalls metallisierten Mono-oder Disazofarbstoff, einem Triphendioxazin, einem Anthrachinon, einem metallisierten Formazan oder einem metallisierten Phthalocyanin ableitet,
- L¹: im Fall 1) eine Azobrücke oder
im Fall 2) ein Brückenglied der Formel O₂S-NZ¹, OC-NZ¹, Z¹N-SO₂, Z¹N-CO, Z¹N-CO-NZ², NZ¹ oder 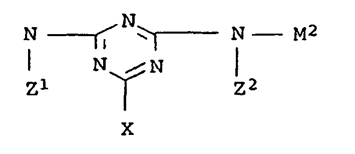 worin M² für eine direkte Bindung oder Methylen, Z¹ und Z² unabhängig voneinander jeweils für Wasserstoff, C₁-C₆-Alkyl oder Phenyl und X für Fluor, Chlor oder Brom, Amino, C₁-C₆-Alkylamino, das gegebenenfalls durch 1 oder 2 nicht benachbarte Sauerstoffatome, Imino- oder C₁-C₄-Alkyliminogruppe unterbrochen und gegebenenfalls mit Hydroxy substituiert ist, N-Pyrrolidinyl, N-Piperidinyl, N-Morpholinyl, N-Piperazinyl oder N-(C₁-C₄)-Alkylpiperazinyl oder NZ¹ oder NZ² auch für Piperazin-1,4-diyl stehen,
- L²: einen Rest der Formel 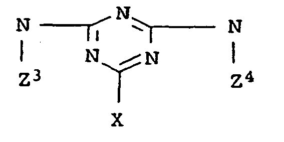 oder 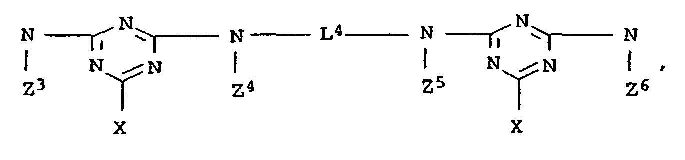 worin Z³, Z⁴, Z⁵ und Z⁶ unabhängig voneinander jeweils für Wasserstoff, C₁-C₆-Alkyl oder Phenyl und L⁴ für C₂-C₈-Alkylen oder gegebenenfalls durch C₁-C₄-Alkyl, C₁-C₄-Alkoxy oder Hydroxysulfonyl substituiertes Phenylen stehen und X jeweils die obengenannte Bedeutung besitzt, bedeuten
zum Färben von Haar, ein Verfahren zur Haarfärbung sowie kos-metische Zubereitungen zum Färben menschlichen von Haaren.

Die Verwendung von Reaktivfarbstoffen, die unter die allgemeine Formel I fallen, zum Färben von Stickstoffatomen enthaltenden Fasern wie Wolle ist beispielsweise aus der US-A-4066 638, EP-A-107 614, EP-A-559 617, DE-A-3 441 273 und DE-A-2 154 942 bekannt. Färberezepturen für Baumwolle und Wolle beinhalten je nach Färbeverfahren pH-Werte von 10 - 12, Temperaturen zwischen 60 und 80°C und Einwirkzeiten um die 10 Stunden.

Aus der US-A-4 102 641 ist die Verwendung von Farbstoffen zum Färben von Haar mit einem Halogentriazin als faserreaktive Gruppe bekannt.

Weiterhin beschreibt die JP-A-75 025 529 Farbstoffformulierungen als Haarfarben. Die hierbei verwendeten Reaktivfarbstoffe gehen von p-Sulfatoethylsulfonylanilin als Diazokomponente und faserreaktivem Rest aus. Um gleichmäßige Färbungen mit diesen Farbstoffen zu erzielen, sind relativ lange Einwirkzeiten notwendig.

EP-A-0181585 beschreibt Reaktivfarbstoffe und deren Verwendung zum Färben von hydroxy-und/oder carbonamidgruppenhaItigem MateriaI, insbesondere Fasermaterial, u. a. Wollfasern. EP-A-0181585 beschreibt auch ein Verfahren zum Färben von Fasern **dadurch gekennzeichnet, daß** die Reaktivfarbstoffe verwendet werden.

DE-A-2154942 beschreibt die Verwendung der Reaktivfarbstoffe der Formel I zum Färben von Fasermaterial, u.a. Wollfasern..

DE-A-19523245 beschreibt Reaktivfarbstoffe und ein Verfahren zum Färben von Hydroxyl-oder Amidgruppen (wie Keratinfasern) enthaltenden Materialien **dadurch gekennzeichnet, daß** die Reaktivfarbstoffe verwendet werden.

EP-A-0437006 beschreibt die Verwendung von Benzophenon als Komponente für kosmetische Zusammensetzungen, die nützlich gegen Bleichung von gefärbten Haaren sind.

EP-A-0433764 und EP-A-0733680 beschreiben Reaktivfarbstoffe die sich von den vorliegenden der Formel I durch die Natur des Susbstituenten des Triazinrestes unterscheiden.

Daher war es Aufgabe der vorliegenden Erfindung, Farbstoffe zum Färben von menschlichen Haaren zur Verfügung zu stellen, die schohnende Färbebedingungen, wie milde pH-Werte, kurze Einwirkzeiten und niedrige Temperaturen ermöglichen.

Demgemäß wurde die Verwendung von Reaktivfarbstoffen der allgemeinen Formel I zum Färben von menschlichen Haaren gefunden.

Die Reaktivfarbstoffe der Formel I sind jeweils in Form der freien Säure angegeben. Selbstverständlich ist auch die Verwendung ihrer physiologisch verträglichen Salze von der vorliegenden Erfindung mit umfaßt.

Hierbei geeignete Kationen leiten sich von Metall- oder Ammoniumionen ab. Metallionen sind insbesondere die Lithium-, Natrium-oder Kaliumionen. Unter Ammoniumionen im erfindungsgemäßen Sinne sind unsubstituierte oder substituierte Ammoniumkationen zu verstehen. Substituierte Ammoniumkationen sind z.B. Monoalkyl-Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-di-alkylsubstituierte Produkte. Unter Alkyl ist dabei im allgemeinen geradkettiges oder verzweigtes C₁-C₂₀-Alkyl zu verstehen, das durch 1 oder 2 Hydroxygruppen substituiert und/oder durch 1 bis 4 Sauerstoffatomen in Etherfunktion unterbrochen sein kann.

Generell können alle, sowohl die obengenannten wie auch in den nachfolgenden Formeln auftretenden Alkyl- und Alkylengruppen sowohl geradkettig als auch verzweigt sein.

Substituierte Alkylreste weisen, sofern nicht anders angegeben, vorzugsweise 1, 2 oder 3 Substituenten, insbesondere 1 oder 2 Substituenten in beliebiger Position auf.

Reste Z¹, Z², Z³, Z⁴, Z⁵ und Z⁶ sind z.B. Methyl, Ethyl, Propyl, Isopropyl, Butyl, Isobutyl, sec.-Butyl, tert.-Butyl, Pentyl, Isopentyl, Neopentyl, tert-Pentyl, Hexyl oder 2-Methylpentyl.

Reste M¹ und L⁴ sind z.B. (CH₂)₂, (CH₂)₃, (CH₂)₄, CH(CH₃)CH₂, CH(CH₃)CH(CH₃), (CH₂)₅ oder (CH₂)₆.

Reste M¹ sind weiterhin (CH₂)₂O(CH₂)₂, (CH₂)₃O(CH₂)₂, (CH₂)₂O(CH₂)₂O(CH₂)₂, (CH₂)₂NH(CH₂)₂, (CH₂)₃NH(CH₂)₂, (CH₂)₂NH(CH₂)₂NH(CH₂)₂,

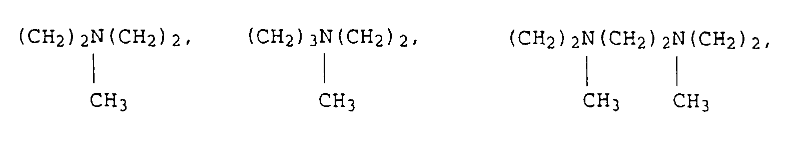

Reste L⁴ sind weiterhin z.B. (CH₂)₇, (CH₂)₈, 1,2-, 1,3- oder 1,4-Phenylen, das jeweils ein- oder zweifach durch Methyl, Methoxy oder Hydroxysulfonyl substituiert sein kann.

Reste X sind Methylamino, Ethylamino, Propylamin, Isopropylamino, Butylamino, Isobutylamin, tert-Butylamino, Pentylamino, Hexylamino, Hydroxymethylamino, 2-Hydroxyethylamino, 2- oder 3-Hydroxypropylamino, 2- oder 4-Hydroxybutylamino, Methoxymethyl amino, 2-Methoxyethylamino, 2- oder 3-Methoxypropylamino, 2- oder 4-Methoxybutylamino, Ethoxymethylamino, Ethoxyethylamino, Ethoxypropylamino, Ethoxybutylamino, Propoxyethylamino oder Propoxypropylamino.

Der Rest Q steht für eine unter alkalischen Reaktionsbedingungen abspaltbare Gruppe. Solche Gruppen sind z.B. Chlor, Brom, C₁-C₄-Alkylsulfonyl, Phenylsulfonyl, OSO₃H, SSO₃H, OP(O)(OH)₂, C₁-C₄-Alkylsulfonyloxy, Phenylsulfonyloxy, C₁-C₄-Alkanoyloxy, C₁-C₄-Dialkylamino oder ein Rest der Formel 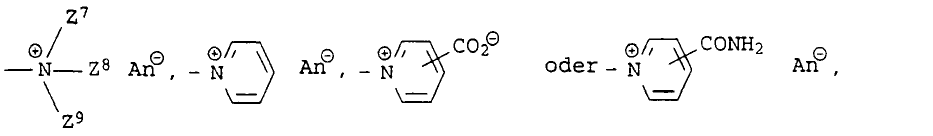 wobei Z⁷, Z⁸ und Z⁹ gleich oder verschieden sind und unabhangig voneinander jeweils die Bedeutung von C₁-C₄-Alkyl oder Benzyl und An^{⊝} jeweils die Bedeutung eines Aquivalents eines Anions besitzen. Als Anionen können dabei z.B. Fluorid, Chlorid, Bromid, Iodid, Mono-, Di- oder Trichloracetat, Methansulfonat, Benzolsulfonat oder 2- oder 4-Methylbenzolsulfonat in Betracht kommen.

Wenn a 2 bedeutet, können die Reste 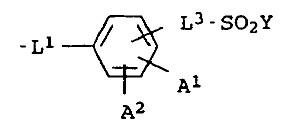 gleich oder verschieden sein.

Wenn b 1 bedeutet, können die Reste W ebenfalls gleich oder verschieden sein.

Der faserreaktive Rest der Formel II 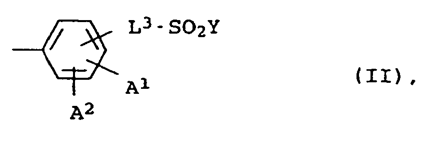 in der A¹, A², L³ und Y jeweils die obengenannte Bedeutung besitzen, wird im folgenden als "E" bezeichnet.

Bevorzugt zum Färben von menschlichen Haaren sind Reaktivfarbstoffe der Formel I, in der L³ eine direkte Bindung bedeutet.

Weiterhin wird die Verwendung von Reaktivfarbstoffen der Formel I, in der A¹ Hydroxysulfonyl bedeutet, bevorzugt.

Ebenfalls ist die Verwendung von Reaktivfarbstoffen der Formel I, in der L³ eine direkte Bindung und A¹ einen Rest der Formel SO₂Y bedeutet, bevorzugt.

Zum Färben von menschlichen Haaren sind außerdem Reaktivfarbstoffe der Formel I, in der A² Wasserstoff oder insbesondere Hydroxysulfonyl bedeutet, bevorzugt.

Weiterhin werden zum Färben von Haaren Reaktivfarbstoffe der Formel I bevorzugt, in der L¹ für eine Azobrücke steht.

Bevorzugt wird ferner die Verwendung von Reaktivfarbstoffen der Formel I, in der L¹ für ein Brückenglied der Formel 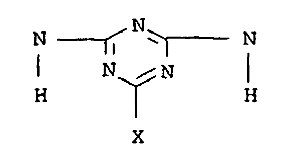 steht und X die obengenannte Bedeutung hat.

Insbesondere wird die Verwendung von Reaktivfarbstoffen der Formel I bevorzugt, in der der Hydroxysulfonylrest in ortho- oder para-Stellung zu L¹ steht und der faserreaktive Rest in para- oder ortho-Stellung zu L¹ oder in para-Stellung zum Hydroxysulfonylrest steht.

Ganz besonders werden Reaktivfarbstoffe der Formel I zum Färben von menschlichen Haaren bevorzugt, deren faserreaktiver Rest den Formel II a - g 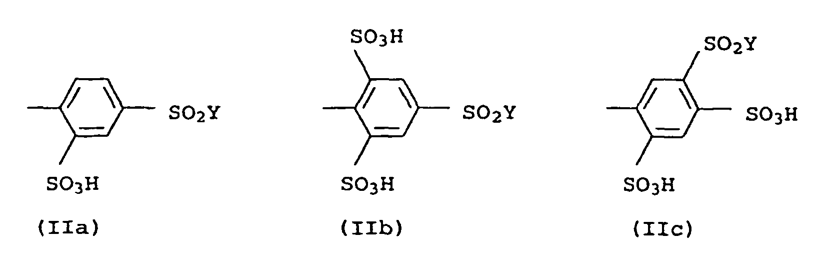 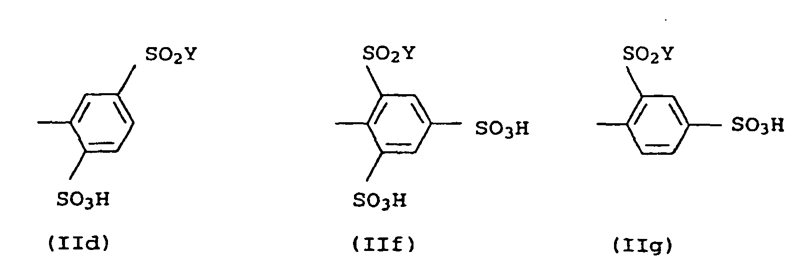 gehorcht.

Weiterhin sind zum Färben von Haaren Reaktivfarbstoffe der Formel I, in der Y für einen Rest der Formel -C₂H₄SSO₃H, -C₂H₄Cl, -C₂H₄OCOCH₃ sowie besonders -C₂H₄OSO₃H oder insbesondere Vinyl steht, bevorzugt.

Daneben wird die Verwendung von Farbstoffen der Formel I bevorzugt, in denen die Substituenten aus einer Kombination der oben aufgeführten bevorzugten Substituenten ausgewahlt sind.

Die Reste SO₂Y sind additiv reagierende faserreaktive Reste, die man von den substitutiv reagierenden faserreaktiven Resten unterscheidet.

Daß die faserreaktive Gruppe mit den betreffenden nucleophilen Gruppen (HNuc-) in den Substraten, z.B. mit den Aminogruppen der Haare, substitutiv reagiert, bedeutet, daß die Austrittsgruppen oder -atome (z.B. Fluor oder Chlor) des faserreaktiven Restes durch die Aminogruppen der Haare gemaß folgendem Schema substituiert werden:

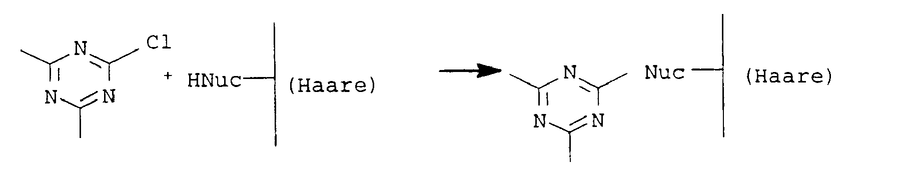

Daß die faserreaktive Gruppe mit den betreffenden Gruppen in den Substraten, z.B. mit den Aminogruppen der Haare, additiv reagiert, bedeutet, daß die Aminogruppen der Haare gemaß folgendem Schema an den faserreaktiven Rest addiert werden:

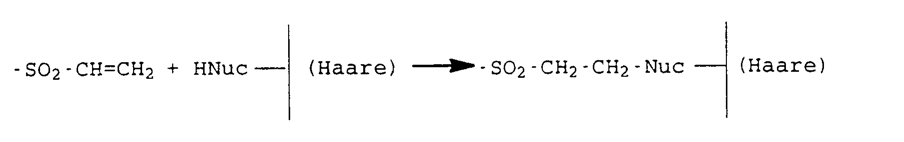

Substitutiv reagierende faserreaktive Reste sind z.B. halogensubstituierte Reste, die sich vom 1,3,5-Triazin, Chinoxalin, Phthalazin, Pyrimidin, Pyridazin oder dem 2-Alkylsulfonylbenzthiazol als heterocyclischen Grundkorper ableiten.

Besonders seien folgende heterocyclische Reste genannt: 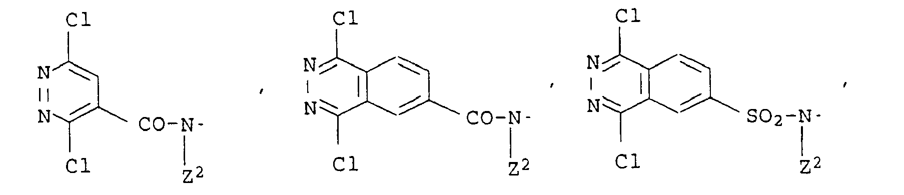 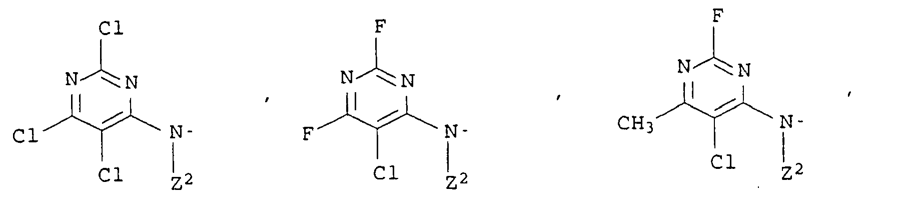 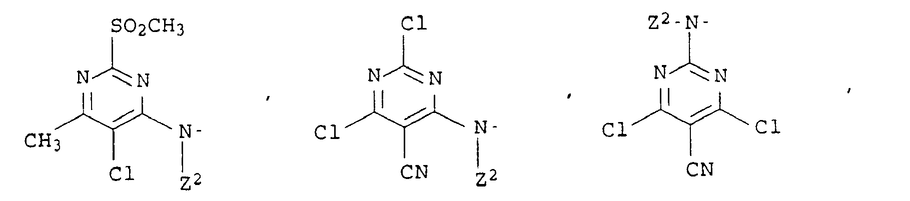 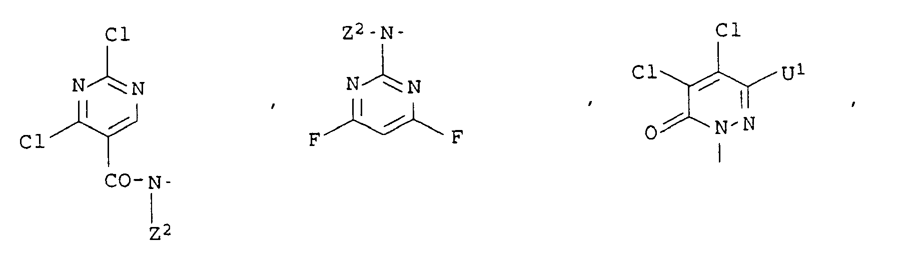 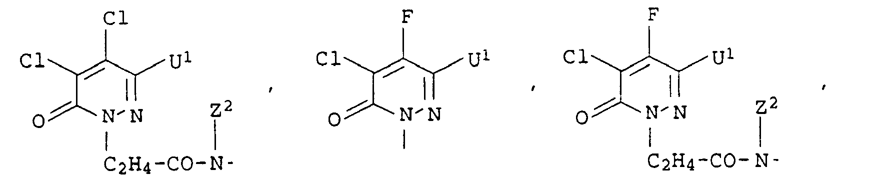 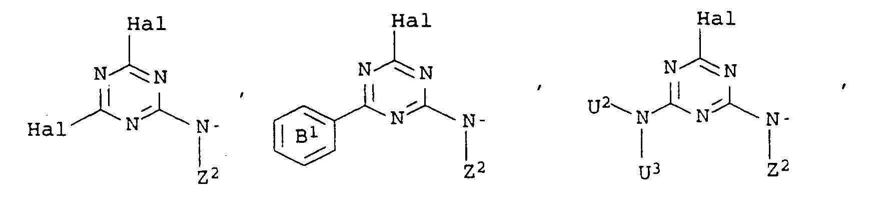 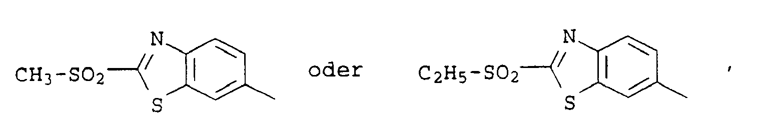 worin Z^{Z} jeweils die obengenannte Bedeutung besitzt und
Hal, Fluor, Chlor oder Brom
- U¹: Wasserstoff oder Nitro und
- U²: und U³ unabhangig voneinander jeweils Wasserstoff, C₁-C₆-Alkyl, das gegebenenfalls durch Hydroxy, Halogen, Cyano, Hydroxysulfonyl oder einen Rest der Formel -SO₂-Y, worin Y die obengenannte Bedeutung besitzt, substituiert ist und jeweils durch 1 oder 2 Sauerstoffatome in Etherfunktion, Imino- oder C₁-C₄-Alkyliminogruppen unterbrochen sein kann, oder
- U²: und U³ zusammen mit dem sie verbindenden Stickstoffatom, Pyrrolidinyl, Piperidinyl, Morpholinyl, Piperazinyl oder N-(C₁-C₄-Alkyl)piperazinyl oder
- U²: auch einen Rest der Formel 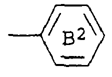 bedeuten,
wobei die Ringe B¹ und B² jeweils ein- oder zweifach durch Hydroxysulfonyl substituiert und/oder benzoanelliert sein können und der Ring B² davon unabhängig ein- oder zweifach durch Chlor, Nitro, C₁-C₄-Alkyl, C₁-C₄-Alkoxy, Cyano, Carboxyl, Acetylamino, Hydroxysulfonylmethyl oder einen Rest der Formel CH₂-SO₂-Y, SO₂-Y, NH-CO-Y oder NU²-CO-NU²-L⁵-SO₂-Y, worin Y und U² jeweils die obengenannte Bedeutung besitzen und L⁵ für C₂-C₆-Alkylen, das gegebenenfalls durch Hydroxy, Chlor, Cyano, Carboxyl, C₁-C₄-Alkoxycarbonyl, C₁-C₄-Alkanoyloxy oder Sulfato substituiert ist und durch jeweils 1 oder 2 Sauerstoffatome in Etherfunktion oder Imino- oder C₁-C₄-Alkyliminogruppen unterbrochen sein kann, steht, substituiert sein kann.

Additiv reagierende faserreaktive Reste sind beispielsweise Acryloyl-, Mono-, Di- oder Trichloracryloyl, Mono-, Di- oder Tribromacryloyl, -CO-CC1=CH-COOH, -CO-CH=CC1-COOH, 2-Chlorpropionyl, 1,2-Dichlorpropionyl, 1,2-Dibrompropionyl, 3-Phenylsulfonylpropionyl, 3-Methylsulfonylpropionyl, 2-Sulfatoethylaminosulfonyl, 2-Chlor-2,3,3-trifluorcyclobutylcarbonyl, 2,2,3,3-Tetrafluorcyclobutylcarbonyl, 2,2,3,3-Tetrafluorcyclobutylsulfonyl, 2-(2,2,3,3-Tetrafluorcyclobutyl)acryloyl, 1- oder 2-Alkyl- oder 1- oder 2-Arylsulfonylacryloyl, wie 1- oder 2-Methylsulfonylacryloyl, oder ein Rest der Formel SO₂-Y, CONH-L⁶-SO₂-Y oder NHCONH-L⁶-SO₂-Y, worin Y die obengenannte Bedeutung besitzt und L⁶ für C₁-C₄-Alkylen oder Phenylen steht.

W in Formel I stellt im Fall 1) z.B. den Rest einer Kupplungskomponente, eines Monoazofarbstoffs oder zusätzlich, wenn b=0, eines Disazofarbstoffs dar, der gegebenenfalls zusätzliche faserreaktive Gruppen aufweist. In diesem Fall ist der Ankerrest E über eine Azobrücke (-N=N-) an den Rest W geknüpft. Handelt es sich bei W um einen Monoazofarbstoff, so ist dessen Kupplungskomponente mit dem Ankerrest E über eine Azobrücke verknüpft. Entsprechend wird, wenn W ein Disazofarbstoff ist, auf seine Diazokomponente gekuppelt.

Zum Färben von menschlichen Haaren geeignete Reaktivfarbstoffe dieser Klasse gehorchen z.B. den Formeln IIIa, IIIb, IIIc oder IIId

(E-N=N-)ₐK (IIIa)

E-N=N-K-N=N-D (IIIb)

E-N=N-(D-N=N-K)-N=N-D (IIIc)

(E-N=N-)₂(K-N=N-D) (IIId)

worin K den Rest einer Kupplungskomponente, D den Rest einer Diazokomponente und a 1 oder 2 bedeuten und E die obengenannte Bedeutung besitzt. Wenn in den Formeln IIIa und IIId der Rest E doppelt auftritt (a=2), können die Reste E entweder gleich oder verschieden voneinander sein. Ebenso können in Formel IIIc die Reste D gleich oder verschieden sein.

Für das Färben von Haaren sind Farbstoffe dieser Klasse wie wasserlösliche Azofarbstoffe, insbesondere Monoazofarbstoffe der Formel IIIa (a=1), Disazofarbstoffe der Formel IIIa (a=2) oder IIIb oder Trisazofarbstoffe der Formeln IIIc oder IIId, die Hydroxysulfonyl- und/oder Carboxylgruppen aufweisen, wertvoll.

Wichtige Kupplungskomponenten HK leiten sich z.B. von Verbindungen aus der Benzol-, Naphthalin-, Pyrazol-, Pyridin-, Pyrimidin-, Indol- oder N-Arylacetoacetamidreihe ab.

Wichtige Diazokomponenten D-NH₂ leiten sich z.B. von Verbindungen aus der Anilin- oder Aminonaphthalinreihe ab. Dabei ist es möglich, sie zugleich als Kupplungskomponente zu verwenden. Insofern sind die Begriffe Diazo- und Kupplungskomponente nicht zwingend für das Herstellverfahren, sondern spiegeln lediglich ein mögliches Verfahren wider.

W in Formel I in Fall 2) stellt weiterhin z.B. den gegebenenfalls metallisierten Rest eines Azofarbstoffs dar. Geeignete Azofarbstoffe, von denen sich solche Reste W ableiten, sind an sich bekannt und in großer Zahl beschrieben, z.B. in K. Venkataraman "The Chemistry of Synthetic Dyes", Vol. VI, Academic Press, New York, London, 1972. Geeignete Azofarbstoffe gehorchen z.B. der Formel IV

D-N=N-K(-N=N-D)₁ (IV),

in der D den Rest einer Diazokomponente, K den Rest einer Kupplungskomponente und 1 0 oder 1 bedeuten, worin, wenn 1 1 ist, die Reste D gleich oder verschieden voneinander sind.

Wertvolle Farbstoffe, von denen sich der Rest W ableitet, sind z.B. wasserlösliche Azofarbstoffe, insbesondere Monoazofarbstoffe der Formel IV (1 = 0), die Hydroxysulfonyl- und/oder Carboxylgruppen aufweisen können.

Bevorzugt leitet sich der Rest W von nichtmetallisierten Azofarbstoffen ab, insbesondere von solchen, die Sulfonsaure- und/oder Carboxylgruppen enthalten, wobei jene, die 1 bis 6 Sulfonsauregruppen aufweisen, besonders hervorzuheben sind.

Wichtige Azofarbstoffe, von denen sich der Rest W sowohl in Fall 1) als auch Fall 2) ableitet, sind beispielsweise solche der Phenyl-azo-naphthalin-, Phenyl-azo-1-phenylpyrazol-5-on-, Phenyl-azo-benzol-, Naphthyl-azo-benzol-, Phenyl-azo-aminonaphthalin-, Naphthyl-azo-naphthalin-, Naphthyl-azo-1-phenylpyrazol-5-on-, Phenyl-azo-pyridon-, Phenyl-azo-aminopyridin-, Naphthyl-azo-pyridon-, Naphthyl-azo-aminopyridin- oder Stilbyl-azo-benzolreihe.

Reste D von Diazokomponenten der Anilin- oder Aminonaphthalinreihe, die keine faserreaktiven Gruppen tragen, leiten sich beispielsweise von Aminen der Formeln Va-f 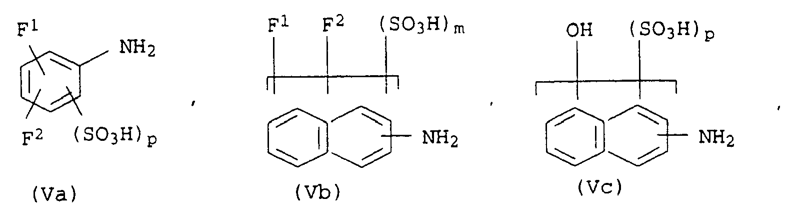 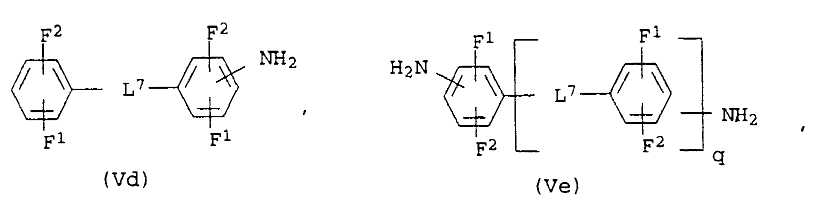 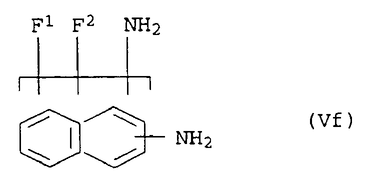 ab, worin
- m: 0, 1, 2 oder 3,
- p: 0, 1 oder 2,
- q: 0 oder 1,
- F¹: Wasserstoff, C₁-C₄-Alkyl, C₁-C₄-Alkoxy, Acetyl, Cyano, Carboxyl, Hydroxysulfonyl, C₁-C₄-Alkoxycarbonyl, Hydroxy, Carbamoyl, Mono- oder Di-(C₁-C₄)-alkylcarbamoyl, Fluor, Chlor, Brom oder Trifluormethyl,
- F²: Wasserstoff, C₁-C₄-Alkyl, C₁-C₄-Alkoxy, Hydroxysulfonylmethyl, Cyano, Carboxyl, Hydroxysulfonyl, Acetylamino, C₁-C₄-Alkoxy-carbonyl, Carbamoyl, Mono- oder Di(C₁-C₄)-alkylcarbamoyl, Fluor, Chlor, Nitro, Sulfamoyl, C₁-C₄-Mono- oder Dialkyl-sulfamoyl, C₁-C₄-Alkylsulfonyl, Phenylsulfonyl oder Phenoxy und
- L⁷: eine direkte Bindung, Sauerstoff, Schwefel oder einen Rest der Formel -NHCO-, -NHCONH-, -CONH-, -CO-, -NHSO₂-, -SO₂NH-, -SO₂-, -CH=CH-, -CH₂-CH₂-, -CH₂-, -NH-, oder -N=N- bedeuten.

Bevorzugt sind dabei solche Komponenten, in denen F¹ Wasserstoff, Methyl, Methoxy, Carboxyl, Hydroxysulfonyl, Hydroxy oder Chlor, F² Wasserstoff, Methyl, Methoxy, Carboxyl, Hydroxysulfonyl, Acetylamino oder Chlor und L⁷ einen Rest der Formel -CO-, -SO₂-, -CH=CH-, -CH₂-CH₂-, -CH₂- oder -N=N- bedeuten.

Aromatische Amine, die sich als Diazokomponenten eignen und die der Formel Va, Vb, Vc oder Vd entsprechen, sind beispielsweise Anilin, 2-Methoxyanilin, 2-Methylanilin, 4-Chlor-2-aminoanisol, 4-Methylanilin, 4-Methoxyanilin, 2-Methoxy-5-methylanilin, 2,5-Dimethoxyanilin, 2,5-Dimethylanilin, 2,4-Dimethylanilin, 2,5-Diethoxyanilin, 2-Chloranilin, 3-Chloranilin, 4-Chloranilin, 2,5-Dichloranilin, 4-Chlor-2-nitroanilin, 4-Chlor-2-methylanilin, 3-Chlor-2-methylanilin, 4-Chlor-2-aminotoluol, 4-Phenylsulfonylanilin, 2-Ethoxy-1-naphthylamin, 1-Naphthylamin, 2-Naphthylamin, 4-Methylsulfonylanilin, 2,4-Dichloranilin-5-carbonsaure, 2-Aminobenzoesaure, 4-Aminobenzoesaure, 3-Aminobenzoesaure, 3-Chloranilin-6-carbonsaure, Anilin-2- oder -3- oder -4-sulfonsaure, Anilin-2,5-disulfonsaure, Anilin-2,4-disulfon-saure, Anilin-3,5-disulfonsaure, 2-Aminotoluol-4-sulfonsaure, 2-Aminoanisol-4-sulfonsaure, 2-Aminoanisol-5-sulfonsaure, 2-Ethoxyanilin-5-sulfonsaure, 2-Ethoxyanilin-4-sulfonsaure, 4-Hydroxysulfonyl-2-aminobenzoesaure, 2,5-Dimethoxyanilin-4-sulfonsaure, 2,4-Dimethoxyanilin-5-sulfonsaure, 2-Methoxy-5-methylanilin-4-sulfonsaure, 4-Aminoanisol-3-sulfonsaure, 4-Aminotoluol-3-sulfonsaure, 2-Aminotoluol-5-sulfonsaure, 2-Chloranilin-4-sulfonsaure, 2-Chloranilin-5-sulfonsaure, 2-Bromanilin-4-sulfonsaure, 2,6-Dichloranilin-4-sulfonsaure, 2,6-Dimethylanilin-3- oder -4-sulfonsaure, 3-Acetylaminoanilin-6-sulfonsaure, 4-Acetyl-aminoanilin-2-sulfonsaure, 1-Aminonaphthalin-4-sulfonsaure, 1-Aminonaphthalin-3-sulfonsaure, 1-Aminonaphthalin-5-sulfonsaure, 1-Aminonaphthalin-6-sulfonsaure, 1-Aminonaphthalin-7-sulfonsaure, 1-Aminonaphthalin-3,7-disulfonsaure, 1-Aminonaphthalin-3,6,8-trisulfonsaure, 1-Aminonaphthalin-4,6,8-trisulfonsaure, 2-Naphthylamin-5- oder -6- oder -8-sulfonsaure, 2-Aminonaphthalin-3,6,8-trisulfonsaure, 2-Aminonaphthalin-6,8-disulfonsaure, 2-Aminonaphthalin-1,6-disulfonsaure, 2-Aminonaphthalin-1-sulfonsaure, 2-Aminonaphthalin-1,5-disulfonsaure, 2-Aminonaphhthalin-3,6-disulfonsaure, 2-Aminonaphthalin-4,8-disulfonsaure, 2-Aminophenol-4-sulfonsaure, 2-Aminophenol-5-sulfonsaure, 3-Aminophenol-6-sulfonsaure, 1-Hydroxy-2-aminonaphthalin-5,8- oder -4,6-disulfonsaure, 4-Aminodiphenylamin, 4-Amino-4'-methoxydiphenylamin, 4-Amino-4'-methoxydiphenyl-amin-3-sulfonsaure, 4-(2'-Methylphenylazo)-2-methylanilin, 4-Aminoazobenzol, 4'-Nitrophenylazo-1-aminonaphthalin, 4-(6'-Hydroxysulfonylnaphthylazo)-1-aminonaphthalin, 4-(2',5'-Dihydroxysulfonylphenylazo)-1-aminonaphthalin, 4'-Amino-3'-methyl-3-nitrobenzophenon, 4-Aminobenzophenon, 4-(4'-Aminophenylazo)-benzolsulfonsaure, 4-(4'-Amino-3'-methoxyphenylazo)benzolsulfonsaure oder 2-Ethoxy-1-naphthylamin-6-sulfonsaure.

Aromatische Diamine, die sich als Tetraazokomponenten oder auch zur Verdoppelung (z.B. mit Cyanurchlorid) eignen und die der Formel Ve oder Vf entsprechen, sind beispielsweise 1,3-Diaminobenzol, 1,3-Diaminobenzol-4-sulfonsaure, 1,4-Diaminobenzol, 1,4-Diaminobenzol-2-sulfonsaure, 1,4-Diamino-2-methylbenzol, 1,4-Diamino-2-methoxybenzol, 1,3-Diamino-4-methylbenzol, 1,3-Diaminobenzol-5-sulfonsaure, 1,3-Diamino-5-methylbenzol, 1,6-Diaminonaphthalin-4-sulfonsaure, 2,6-Diaminonaphthalin-4,8-disulfonsaure, 3,3'-Diaminodiphenylsulfon, 4,4'-Diaminodiphenylsulfon, 4,4'-Diaminostilben-2,2'-disulfonsaure, 2,2'-Diaminodiphenylsulfon, 2,2'-Diaminodiphenylsulfon-4,5-disulfonsaure, 4,4'-Diaminobenzophenon, 4,4'-Diamino-3,3'-dinitrobenzophenon, 3,3'-Diamino-4,4'-dichlorbenzophenon, 4,4'- oder 3,3'-Diamino diphenyl, 4,4'-Diamino-3,3'-dichlordiphenyl, 4,4'-Diamino-3,3'-dimethoxy- oder -3,3'-dimethyl- oder -2,2'-dimethyl- oder -2,2'-dichlor- oder -3,3'-diethoxydiphenyl, 4,4'-Diamino-3,3'-dimethyl-6,6'-dinitrodiphenyl, 4,4'-Diaminodiphenyl-2,2'- oder -3,3'-disulfonsäure, 4,4'-Diamino-3,3'-dimethyl- oder -3,3'-dimethoxy- oder -2,2'-dimethoxydiphenyl-6,6'-disulfonsaure, 4,4'-Diamino-2,2',5,5'-tetrachlordiphenyl, 4,4'-Diamino-3,3'-di-nitrodiphenyl, 4,4'-Diamino-2,2'-dichlor-5,5'-dimethoxydiphenyl, 4,4'-Diaminodiphenyl-2,2'- oder -3,3'-dicarbonsaure, 4,4'-Diamino-3,3'-dimethyldiphenyl-5,5'-disulfonsaure, 4,4'-Diamino-2-nitrodiphenyl, 4,4'-Diamino-3-ethoxy- oder -3-hydroxysulfonyl-diphenyl, 4,4'-Diamino-3,3'-dimethyldiphenyl-5-sulfonsaure, 4,-4'-Diaminodiphenylmethan, 4,4'-Diamino-3,3'-dimethyldiphenyl-methan, 4,4'-Diamino-2,2',3,3'-tetramethyldiphenylmethan, 4,4'-Diaminodiphenylethan, 4,4'-Diaminostilben oder 4,4'-Diaminodiphenylmethan-3,3'-dicarbonsaure.

Aromatische Reste D von Diazokomponenten aus der Anilin- oder Aminonaphthalinreihe, die weitere faserreaktive Reste tragen, leiten sich beispielsweise von Aminen der Formeln VIa-c 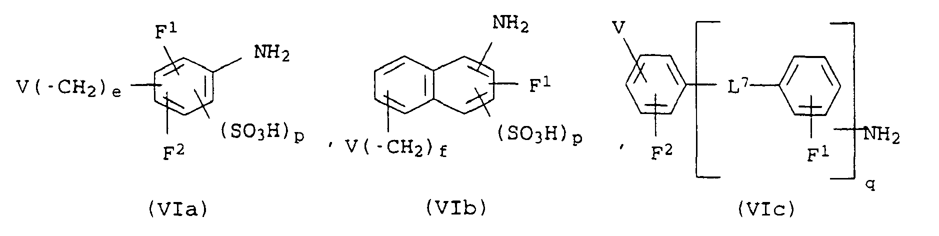 ab, in denen F¹, F², p, q und L⁷ jeweils die obengenannte Bedeutung besitzen und e und f gleich oder verschieden sind und unabhängig voneinander jeweils 0 oder 1 und V einen faserreaktiven Rest bedeuten.

Faserreaktive Reste V leiten sich beispielsweise vom Rest E ab oder sind, wie oben bereits ausgefuhrt, weitere additiv reagierende oder substitutiv reagierende faserreaktive Reste.

Aromatische Amine, die den faserreaktiven Rest V aufweisenden Derivaten der Formel VIa, VIb oder VIc zugrundeliegen, sind beispielsweise 1,3-Diaminobenzol, 1,3-Diaminobenzol-4-sulfonsaure, 1,3-Diaminobenzol-4,6-disulfonsaure, 1,4-Diaminobenzol, 1,4-Diaminobenzol-2-sulfonsaure, 1,4-Diaminobenzol-2,5-disulfonsaure, 1,4-Diamino-2-methylbenzol, 1,4-Diamino-2-methoxybenzol, 1,3-Diamino-4-methylbenzol, 1,4-Diaminobenzol-2,6-disulfonsaure, 1,5-Diamino-4-methylbenzol-2-sulfonsaure, 1,5-Diamino-4-methoxybenzol-2-sulfonsaure, 1,6-Diamino-naphth-2-ol-4-sulfonsaure, 1,6-Diaminonaphthalin-4-sulfonsaure, 2,6-Diaminonaphthalin-4,8-disulfonsaure, 2,6-Diaminonaphth-1-ol-4,8-disulfonsaure, 1,3-Diaminobenzol-5-sulfonsäure, 1,3-Diamino-5-methylbenzol, 2,6-Diaminophenol-4-sulfonsaure, 5-Aminomethyl-2-aminonaphthalin-1-sulfonsäure, 5-(N-Methylaminomethyl)-2-aminonaphthalin-1-sulfonsaure, 4,4'-Diaminostilben-3,3'-di-carbonsaure, 4-(N-Methylaminomethyl)anilin-2-sulfonsaure oder 3-(N-Methylaminomethyl)-anilin-6-sulfonsaure.

Die Reste K der Kupplungskomponente entstammen vorzugsweise der Benzol-, Naphthalin-, Pyrazol-, Pyridin-, Pyrimidin-, Indol-oder N-Arylacetoacetamidreihe und können auch faserreaktive Gruppen tragen.

Faserreaktivgruppenfreie Kupplungskomponenten sind bevorzugt Verbindungen der Naphthalin-, Anilin-, Pyrazolon-, Aminopyrazol-, 2,6-Diaminopyridin-, Pyridon-, Hydroxypyrimidin-, Indol-, N-Arylacetoacetamidreihe und entsprechen beispielsweise den Verbindungen der Formeln VII a-m 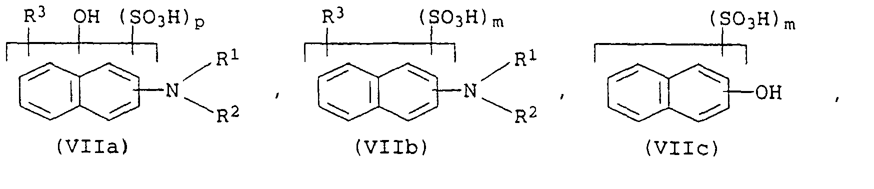 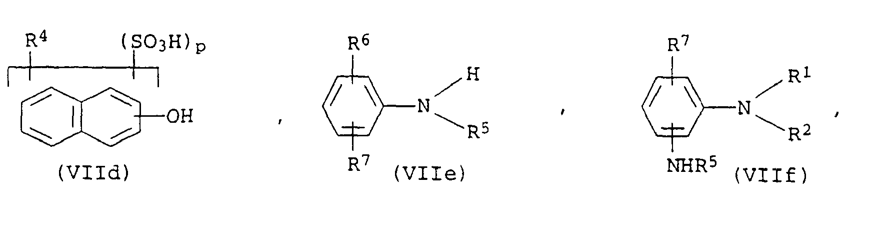 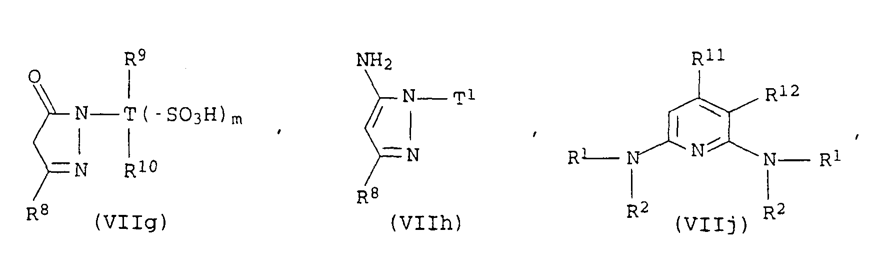 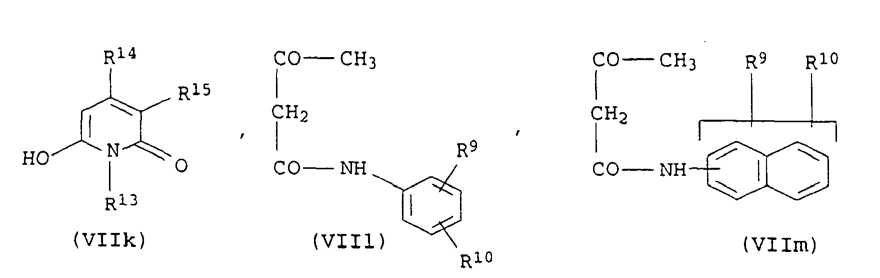 worin
- m: 0, 1, 2 oder 3,
- p: 0, 1 oder 2,
- R¹: Wasserstoff oder C₁-C₄-Alkyl, das durch Hydroxy, Cyano, Carboxyl, Hydroxysulfonyl, Sulfato, Methoxycarbonyl, Ethoxy-carbonyl oder Acetoxy substituiert sein kann,
- R²: Wasserstoff, C₁-C₄-Alkyl, das durch Hydroxy, Cyano, Carboxyl, Hydroxysulfonyl, Sulfato, Methoxycarbonyl, Ethoxycarbonyl oder Acetoxy substituiert sein kann, Benzyl oder Phenyl, das durch C₁-C₄-Alkyl, C₁-C₄-Alkoxy, Chlor oder Hydroxysulfonyl substituiert sein kann,
- R³: Wasserstoff oder C₁-C₄-Alkyl, das durch Hydroxysulfonyl oder Carboxyl substituiert sein kann,
- R⁴: C₁-C₆-Alkylureido, Phenylureido, das durch Chlor, Methyl, Methoxy, Nitro, Hydroxysulfonyl oder Carboxyl substituiert sein kann, C₁-C₆-Alkanoylamino, das durch Hydroxysulfonyl oder Chlor substituiert sein kann, Cyclohexylcarbonylamino, Benzoylamino, das durch Chlor, Methyl, Methoxy, Nitro, Hydroxysulfonyl oder Carboxyl substituiert sein kann, oder Hydroxy,
- R⁵: Wasserstoff, C₁-C₆-Alkyl, insbesondere C₁-C₄-Alkyl, das jeweils durch Phenyl, C₁-C₄-Alkoxy, Hydroxy, Phenoxy oder C₁-C₄-Alkanoyloxy substituiert sein kann, C₅-C₇-Cycloalkyl, Hydroxysulfonylphenyl, C₁-C₄-Alkanoyl, Carbamoyl, Mono- oder Di-(C₁-C₄)-alkylcarbamoyl, Phenylcarbamoyl oder Cyclohexyl-carbamoyl,
- R⁶: C₁-C₄-Alkoxy, Chlor, Brom, Hydroxysulfonyl, C₁-C₄-Alkanoyl-amino, Amino, Ureido, Methylsulfonylamino, Ethylsulfonyl-amino, Dimethylaminosulfonylamino, Methylamino, Ethylamino, Dimethylamino oder Diethylamino
- R⁷: Wasserstoff, C₁-C₄-Alkyl, C₁-C₄-Alkoxy, Hydroxysulfonyl, Chlor oder Brom,
- T: den Rest eines Benzol- oder Naphthalinrings,
- T¹: C₁-C₄-Alkyl, Cyclohexyl, Benzyl oder Phenyl, das ein- bis dreifach durch Fluor, Chlor, Brom, Methoxy, Nitro, Hydroxy-sulfonyl, Carboxyl, Acetyl, Acetylamino, Methylsulfonyl, Sulfamoyl oder Carbamoyl substituiert ist,
- R⁸: Methyl, Carboxyl, C₁-C₄-Alkoxycarbonyl oder Phenyl,
- R⁹: Wasserstoff, C₁-C₄-Alkyl, C₁-C₄-Alkoxy, Acetyl, Cyano, Carboxyl, Hydroxysulfonyl, C₁-C₄-Alkoxycarbonyl, Hydroxy, Carbamoyl, Mono- oder Di-(C₁-C₄)-alkylcarbamoyl, Fluor, Chlor, Brom oder Trifluormethyl,
- R¹⁰: Wasserstoff, C₁-C₄-Alkyl, C₁-C₄-Alkoxy, Cyano, Carboxyl, Hydroxysulfonyl, Acetylamino, C₁-C₄-Alkoxycarbonyl, Carbamoyl, Mono- oder Di-(C₁-C₄)-alkylcarbamoyl, Fluor, Chlor, Nitro, Sulfamoyl, Mono- oder Di-(C₁-C₄)-alkylsulfamoyl, C₁-C₄-Alkyl-sulfonyl, Phenylsulfonyl oder Phenoxy,
- R¹¹: Wasserstoff oder C₁-C₄-Alkyl, das durch C₁-C₄-Alkoxy oder Cyano substituiert sein kann,
- R¹²: Wasserstoff, Methyl, Hydroxysulfonylmethyl, Hydroxysulfonyl, Cyano oder Carbamoyl,
- R¹³: Wasserstoff, C₁-C₄-Alkyl, das durch Phenyl, Hydroxysulfonylphenyl, Hydroxy, Amino, C₁-C₄-Alkoxyl, Carboxyl, Hydroxysulfonyl, Acetylamino, Benzoylamino oder Cyano substituiert sein kann, Cyclohexyl, Phenyl, das gegebenenfalls durch Carboxyl, Hydroxysulfonyl, Benzoylamino, Acetylamino, Methyl, Methoxy, Cyano oder Chlor substituiert ist, oder Amino, das durch Phenyl, C₁-C₄-Alkyl, C₁-C₄-Alkanoyl oder Benzoyl substi tuiert ist,
- R¹⁴: Wasserstoff, C₁-C₄-Alkyl, Phenyl, Hydroxyl, Cyano, Acetyl, Benzoyl, Carboxyl, Methoxycarbonyl, Carbamoyl oder Hydroxysulfonylmethyl und
- R¹⁵: Wasserstoff, Chlor, Brom, Acetylamino, Amino, Nitro, Hydroxysulfonyl, Sulfamoyl, Methylsulfonyl, Phenylsulfonyl, Carboxyl, C₁-C₄-Alkoxycarbonyl, C₁-C₄-Alkanoyl, Benzoyl, Carbamoyl, Cyano oder Hydroxysulfonylmethyl bedeuten.

Reste U², U³, F¹, F², R¹, R², R³, R⁵, R⁷, T¹, R⁹, R¹⁰, R¹¹, R¹³, R¹⁴ sowie die unten erwähnten Reste G³, G⁵, G¹² und G¹³ sind z.B. Methyl, Ethyl, Propyl, Isopropyl, Butyl, Isobutyl, sec-Butyl oder tert-Butyl.

Die Reste U², U³ und R⁵ können weiterhin Pentyl, Isopentyl, Neopentyl, tert-Pentyl, Hexyl oder 2-Methylpentyl sein.

Die Reste U², U³, R¹, R², R⁵ und R¹³ sind Hydroxy-C₁-C₄-alkyl wie Hydroxymethyl, 1-Hydroxyeth-1-yl, 2-Hydroxyeth-1-yl, 1-Hydroxyprop-1-yl, 2-Hydroxyprop-1-yl, 3-Hydroxyprop-1-yl, 1-Hydroxyprop-2-yl, 2-Hydroxyprop-2-yl, 1-Hydroxbut-1-yl, 2-Hydroxybut-1-yl, 3-Hydroxybut-1-yl, 4-Hydroxybut-1-yl, 1-Hydroxybut-2-yl, 2-Hydroxybut-2-yl, 1-Hydroxybut-3-yl, 2-Hydroxybut-3-yl, 1-Hydroxy-2-methyl-prop-3-yl, 2-Hydroxy-2-methylprop-3-yl, 3-Hydroxy-2-methyl-prop-3-yl und 2-Hydroxymethylprop-2-yl.

Die Reste U², U³, R¹, R², R¹¹ und R¹³ sind weiterhin z.B. Cyanomethyl, Cyanoethyl, Cyanopropyl oder Cyanobutyl.

Die Reste R¹, R², R³ und R¹³ sind beispielsweise Carboxylmethyl, Carboxylethyl, 2- oder 3-Carboxylpropyl oder 2- oder 4-Carboxylbutyl.

Die Reste U², U³, R¹, R² und R³ stehen ferner für beispielsweise Hydroxysulfonylmethyl, 2-Hydroxysulfonylethyl, 2- oder 3-Hydroxysulfonylpropyl oder 2- oder 4-Hydroxysulfonylbutyl.

R¹ und R² stehen weiterhin für z.B. 2-Sulfatoethyl, 2- oder 3-Sulfatopropyl, 2- oder 4-Sulfatobutyl, Methoxycarbonylmethyl, 2-Methoxycarbonylethyl, 2- oder 3-Methoxycarbonylpropyl, 2- oder 4-Methoxycarbonylbutyl, Ethoxycarbonylmethyl, 2-Ethoxycarbonylethyl, 2- oder 3-Ethoxycarbonylpropyl, 2- oder 4-Ethoxycarbonylbutyl, Acetoxymethyl, 2-Acetoxyethyl, 2- oder 3-Acetoxypropyl oder 2- oder 4-Acetoxybutyl.

Die Reste R² können weiterhin z.B. 2-, 3- oder 4-Methylphenyl, 2-, 3- oder 4-Ethylphenyl, 2-, 3- oder 4-Propylphenyl, 2-, 3-oder 4-Isopropylphenyl, 2-, 3- oder 4-Butylphenyl, 2-, 3- oder 4-Isobutylphenyl, 2-, 3- oder 4-sec-Butylphenyl, 2-, 3- oder 4-tert-Butylphenyl, 2-, 3- oder 4-Methoxyphenyl, 2-, 3- oder 4-Ethoxyphenyl, 2-, 3- oder 4-Propoxyphenyl, 2-, 3- oder 4-Isopropoxyphenyl, 2-, 3- oder 4-Butoxyphenyl, 2-, 3- oder 4-Isobutoxyphenyl, 2-, 3- oder 4-sec-Butoxyphenyl, 2-, 3- oder 4-tert-Butoxyphenyl oder 2-, 3- oder 4-Chlorphenyl sein.

Die Reste R², R⁵ und T¹ sind weiterhin z.B. 2-, 3- oder 4-Hydroxysulfonylphenyl.

Der Rest R⁴ ist z.B. Methylureido, Ethylureido, Propylureido, Butylureido, Pentylureido, Hexylureido, Formylamino, Acetylamino, Propionylamino, Butyrylamino, Isopropylcarbonylamino, Valerylamino, Isobutylcarbonylamino, sec-Butylcarbonylamino, tert-Butylcarbonylamino oder Pentylcarbonylamino.

Die Reste R⁵ und R¹³ können z.B. Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenyl-but-1-yl, 1-Phenylbut-2-yl, 2-Phenylbut-2-yl, 3-Phenylbut-2-yl, 3-Phenylbut-2-yl, 4-Phenylbut-2-yl, 1-(Phenylmethyl)-eth-1-yl, 1-(Phenylmethyl)-1-(methyl)-eth-1-yl und 1-(Phenylmethyl)-prop-1-yl, vorzugsweise Benzyl und 2-Phenylethyl sein.

Die Reste R⁵, R¹¹ und R¹³ stehen weiter für beispielsweise Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)-propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(n-Propoxy)propyl, 3-(1-Methylethoxy)propyl, 3-(n-Butoxy)- propyl, 3-(1-Methylpropoxy)propyl, 3-(2-Methylpropoxy)-propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl.

Reste R⁵ sind ferner z.B. Phenoxymethyl, 2-Phenoxyethyl, 2-oder 3-Phenoxypropyl, 2- oder 4-Phenoxybutyl, Formyloxymethyl, 2-(Formyloxy)ethyl, 2- oder 3-(Formyloxy)propyl, 2- oder 4-(Formyloxy)butyl, Methylcarbonyloxymethyl, 2-(Methylcarbonyloxy)ethyl, 2- oder 3-(Methylcarbonyloxy)propyl, 2 oder 4-(Methylcarbonyloxy)butyl, Ethylcarbonyloxymethyl, 2-(Ethylcarbonyloxy)ethyl, 2- oder 3-(Ethylcarbonyloxy)propyl, 2- oder 4-(Ethylcarbonyloxy)butyl, Propylcarbonyloxymethyl, 2-(Propylcarbonyloxy)ethyl, 2- oder 3-(Propylcarbonyloxy)propyl, 2- oder 4-(Propylcarbonyloxy)butyl, Cyclopentyl, Cyclohexyl oder Cycloheptyl.

Die Reste R⁵, R¹⁵ sowie die unten beschriebenen Reste G⁴ sind z.B. Formyl, Acetyl, Propionyl, Butyryl oder Isobutyryl.

Die Reste F¹, F², R⁵, R⁹, R¹⁰ sowie die unten beschriebenen Reste G⁴ sind beispielsweise Mono- oder Dimethylcarbamoyl, Mono-oder Diethylcarbamoyl, Mono- oder Dipropylcarbamoyl oder Mono-oder Dibutylcarbamoyl.

Reste F¹, F², R⁶, R⁷, R⁹, R¹⁰ sowie die unten beschriebenen Reste G³ und G⁵ können z.B. Methoxy, Ethoxy, Propoxy, Isopropoxy, Butoxy, sec-Butoxy, Isobutoxy oder tert-Butoxy sein.

Die Reste R⁶ und R¹³ sind beispielsweise Formylamino, Methylcarbonylamino, Ethylcarbonylamino, Propylcarbonylamino oder Isopropylcarbonylamino.

Reste T¹ sind weiterhin z.B. 2-, 3- oder 4-Fluorphenyl, 2-, 3-oder 4-Chlorphenyl, 2-, 3- oder 4-Bromphenyl, 2-, 3- oder 4-Methoxyphenyl, 2-, 3- oder 4-Nitrophenyl, 2-, 3- oder 4-Carboxylphenyl, 2-, 3- oder 4-Acetylphenyl, 2-, 3- oder 4-Acetylaminophenyl, 2-, 3- oder 4-Methylsulfonylphenyl, 2-, 3- oder 4-Sulfamoylphenyl oder 2-, 3- oder 4-Carbamoylphenyl.

Die Reste F¹, F², R⁸, R⁹, R¹⁰, R¹⁵ sowie die unten beschriebenen Reste R¹⁶ sind ferner z.B. Methoxycarbonyl, Ethoxycarbonyl, Propoxycarbonyl, Isopropoxycarbonyl, Butoxycarbonyl, Isobutoxycarbonyl, sec-Butoxycarbonyl oder tert-Butoxycarbonyl.

Die Reste F² und R¹⁰ sind weiterhin beispielsweise Mono- oder Dimethylsulfamoyl, Mono- oder Diethylsulfamoyl, Mono- oder Dipropylsulfamoyl, Mono- oder Dibutylsulfamoyl, Methylsulfonyl, Ethylsulfonyl, Propylsulfonyl oder Butylsulfonyl.

Als Reste R¹³ kommen ferner in Betracht z.B. Hydroxysulfonylphenylmethyl, 2-Hydroxysulfonylphenylethyl, 2- oder 3-Hydroxysulfonylphenylpropyl, 2- oder 4-Hydroxysulfonylphenylbutyl, Aminomethyl, 2-Aminoethyl, 2- oder 3-Aminopropyl, 2- oder 4-Aminobutyl, Hydroxysulfonylmethyl, 2-Hydroxysulfonylethyl, 2-oder 3-Hydroxysulfonylpropyl, 2- oder 4-Hydroxysulfonylbutyl, Acetylaminomethyl, 2-Acetylaminoethyl, 2- oder 3-Acetylaminopropyl, 2- oder 4-Acetylaminobutyl, Benzoylaminomethyl, 2-Benzoylaminoethyl, 2- oder 3-Benzoylaminopropyl, 2- oder 4-Benzoylaminobutyl, 2-, 3- oder 4-Carboxylphenyl, 2-, 3- oder 4-Hydroxysulfonylphenyl, 2-, 3- oder 4-Benzoylaminophenyl, 2-, 3-oder 4-Acetylaminophenyl, 2-, 3- oder 4-Methylphenyl, 2-, 3- oder 4-Methoxyphenyl, 2-, 3- oder 4-Cyanophenyl, 2-, 3- oder 4-Chlorphenyl, Phenylamino, Methylamino, Ethylamino, Propylamino, Isopropylamino, Butylamino, Isobutylamino, sec-Butylamino, tert-Butylamino oder Benzoylamino.

Die Reste L⁵ sowie die unten beschriebenen Reste L⁸ sind z.B. (CH₂)₂, (CH₂)₃, (CH₂)₄, CH(CH₃)CH₂, CH(CH₃)CH(CH₃), (CH₂)₅ oder (CH₂)₆.

Die Reste L⁵ sind weiterhin z.B. (CH₂)₂O(CH₂)₂, (CH₂)₃O(CH₂)₂, (CH₂)₂O(CH₂)₂O(CH₂)₂, (CH₂)₂S(CH₂)₂, (CH₂)₃S(CH₂)₂, (CH₂)₂S(CH₂)₂S(CH₂)₂, (CH₂)₂NH(CH₂)₂, (CH₂)₃NH(CH₂)₂, (CH₂)₂NH(CH₂)₂NH(CH₂)₂,

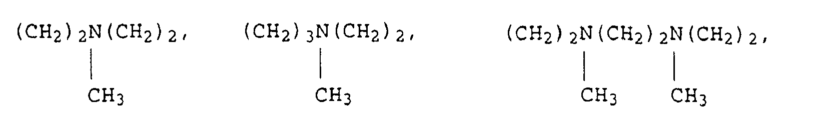

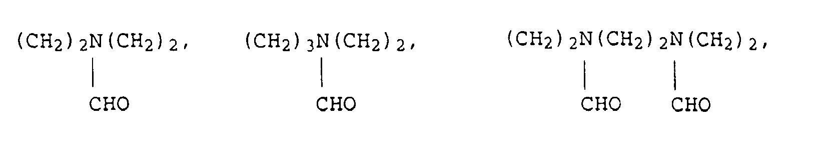

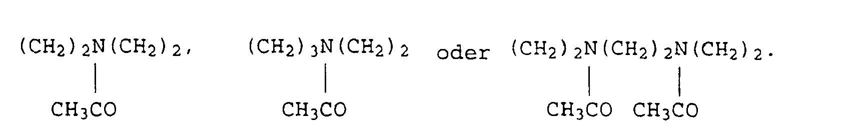

Die Reste L⁶ und L¹¹ sind CH₂, (CH₂)₂, (CH₂)₃, (CH₂)₄, CH(CH₃)CH₂ oder CH(CH₃)CH(CH₃).

Im folgenden werden beispielhaft Kupplungskomponenten KH aufgefuhrt. Im einzelnen sind beispielsweise als Naphtholsulfonsauren 1-Naphthol-3-sulfonsäure, 1-Naphthol-4-sulfonsäure, 1-Naphthol-5-sulfonsäure, 1-Naphthol-8-sulfonsäure, 1-Naphthol-3,6-disulfonsäure, 1-Naphthol-3,8-disulfonsäure, 2-Naphthol-5-sulfonsäure, 2-Naphthol-6-sulfonsäure, 2-Naphthol-7-sulfonsäure, 2-Naphthol-8-sulfonsäure, 2-Naphthol-3,6-disulfonsäure, 2-Naphthol-6,8-disulfonsäure, 2-Naphthol-3,6,8-trisulfonsäure, 1,8-Dihydroxynaphthalin-3,6-disulfonsäure, 2,6-Dihydroxynaphthalin-8-sulfonsäure oder 2,8-Dihydroxynaphthalin-6-sulfonsäure zu nennen.

Weiterhin sind beispielsweise 1-Naphthylamin, N-Phenyl-1-naphthylamin, N-Ethyl-1-naphthylamin, N-Phenyl-2-naphthylamin, 1-Naphthol, 2-Naphthol, 1,5-Dihydroxynaphthalin, 1,6-Dihydroxynaphthalin, 1,7-Dihydroxynaphthalin oder 2,7-Dihydroxynaphthalin zu nennen.

Aminonaphthalinsulfonsauren sind beispielsweise 1-Naphthylamin-6-sulfonsäure, 1-Naphthylamin-7-sulfonsäure, 1-Naphthylamin-8-sulfonsäure, 2-Naphthylamin-3,6-disulfonsäure, 2-Naphthylamin-5,7-disulfonsäure, 2-Naphthylamin-6,8-disulfonsäure, 2-Hydroxysulfonylmethylaminonaphthalin-5-sulfonsäure oder 2-Hydroxysulfonylmethylaminonaphthalin-6-sulfonsäure.

Als Aminonaphtholsulfonsäuren sind z.B. 1-Amino-5-hydroxynaphthalin-7-sulfonsäure, 1-Amino-8-hydroxynaphthalin-4-sulfonsäure, 1-Amino-8-hydroxynaphthalin-2,4-disulfonsäure, 1-Amino-8-hydroxynaphthalin-3,6-disulfonsäure, 1-Amino-8-hydroxynaphthalin-4,6-disulfonsäure, 2-Amino-5-hydroxynaphthalin-7-sulfonsäure, 2-Amino-8-hydroxynaphthalin-6-sulfonsäure, 2-Amino-8-hydroxynaphthalin-3,6-disulfonsäure, 2-Amino-5-hydroxynaphthalin-1,7-disulfonsäure, 3-Amino-7-hydroxysulfonylmethyl-8-hydroxynaphthalin-3,6-disulfonsäure, 2-Amino-7-hydroxysulfonylmethyl-8-hydroxynaphthalin-6-sulfonsäure, 1-Acetylamino-8-hydroxynaphthalin-3,6-disulfonsäure, 1-Benzoylamino-8-hydroxynaphthalin-3,6-disulfonsäure, 1-Acetylamino-8,hydroxynaphthalin-4,6-disulfonsäure, 1-Benzoylamino-8-hydroxynaphthalin-4,6-disulfonsäure, 1-Acetylamino-5-hydroxynaphthalin-7-sulfonsäure, 2-Methylamino-8-hydroxynaphthalin-6-sulfonsäure, 2-Methylamino-8-hydroxynaphthalin-6-sulfonsäure, 2-Hydroxysulfonylmethyl-amino-8-hydroxynaphthalin-6-sulfonsaure, 2-Hydroxysulfonylmethyl-amino-7-hydroxysulfonylmethyl-8-hydroxynaphthalin-6-sulfonsaure, 2-Hydroxysulfonylmethylamino-8-hydroxynaphthalin-3,6-disulfon-saure, 2-Hydroxysulfonylmethylamino-7-hydroxysulfonylmethyl-8-hydroxynaphthalin-3,6-disulfonsaure, 2-(3'- oder 4'-Hydroxysulfonylphenylamino)-8-hydroxynaphthalin-6-sulfonsäure, 2-Acetylamino-5-hydroxynaphthalin-7-sulfonsäure oder 2-Acetylamino-8-hydroxynaphthalin-6-sulfonsaure zu nennen.

Als Benzol-Kupplungskomponenten sind beispielsweise o- oder m-Toluidin, o- oder m-Anisidin, Kresidin, 2,5-Dimethylanilin, 2,5-Dimethoxyanilin, m-Ammoacetanilid, 3-Amino-4-methoxyacet-anilid, 3-Amino-4-methylacetanilid, m-Aminophenylharnstoff, N-Methylanilin, N-Methyl-m-toluidin, N-Ethylanilin, N-Ethyl-m-toluidin, N-(2-Hydroxyethyl)anilin oder N-(2-Hydroxyethyl)-m-toluidin zu nennen.

Als Pyrazolon-Kupplungskomponenten sind beispielsweise 3-Methyl-, 3-Carboxy- oder 3-(C₁-C₄-Alkoxycarbonyl)pyrazol-5-one zu nennen, die in 1-Stellung Wasserstoff, gegebenenfalls durch Methyl, Ethyl, Fluor, Chlor, Brom, Trifluormethyl, Methoxy, Ethoxy, Cyano, Phenoxy, Phenylsulfonyl, Methylsulfonyl, Hydroxysulfonyl, Acetylamino, Nitro, Hydroxyl, Carboxyl, Carbamoyl oder Sulfamoyl substituiertes Phenyl oder durch Hydroxysulfonyl substituiertes 1- oder 2-Naphthyl tragen können. Beispielsweise sind 1-Phenyl-, 1-(2'-Chlorphenyl)-, 1-(2'-Methoxyphenyl)-, 1-(2'-Methylphenyl)-, 1-(1',5'-Dichlorphenyl)-, 1-(2',6'-Dichlorphenyl)-, 1-(2'-Methyl-6'-chlorphenyl)-, 1-(2'-Methoxy-5'-methylphenyl)-, 1-(2'-Methoxy-5'-hydroxysulfonylphenyl)-, 1-(2',5'-Dichlor-4'-hydroxysulfonylphenyl)-, 1-(2',5'-Dihydroxysulfonylphenyl)-, 1-(2'-Carboxyphenyl)-, 1-(3'-Hydroxysulfonylphenyl)-, 1-(4'-Hydroxysulfonylphenyl)- oder 1-(3'-Sulfamoylphenyl)-3-carboxylpyrazol-5-on, 1-(3'- oder 4'-Hydroxysulfonylphenyl)-, 1-(2'-Chlor-4'- oder -5'-hydroxysulfonylphenyl)-, 1-(2'-Methyl-4'-hydroxysulfonylphenyl)-, 1-(2',5'-Dichlorphenyl)-, 1-(4',8'-Dihydroxysulfonyl-1-naphthyl)-, 1-(6'-Hydroxysulfonyl-1-naphthyl)-3-methyl-pyrazol-5-on, 1-Phenylpyrazol-5-on-3-carbonsaureethylester, Pyrazol-5-on-3-carbonsaureethylester oder Pyrazol-5-on-3-carbonsaure zu nennen.

Andere aus der Pyrazolreihe stammende Kupplungskomponenten sind beispielsweise 1-Methyl-, 1-Ethyl-, 1-Propyl-, 1-Butyl-, 1-Cyclohexyl-, 1-Benzyl- oder 1-Phenyl-5-aminopyrazol, 1-(4'-Chlorphenyl)-, 1-(4'-Methylphenyl)-5-aminopyrazol oder 1-Phenyl-3-methyl-5-aminopyrazol.

N-Arylacetoacetamide sind vor allem Acetessiganilid oder dessen im Phenylkern durch Chlor, Methyl, Ethyl, Methoxy, Ethoxy, Acetylamino, Hydroxylsulfonyl, Carboxyl, Carbamoyl oder Sulfamoyl ein- oder mehrfach substituierte Derivate.

Vom Pyridin abgeleitete Kupplungskomponenten sind beispielsweise die in der DE-A-2 260 827 beschriebenen Derivate.

Als Pyrimidinkupplungskomponenten sind z.B. die in der DE-A-2 202 820, DE-A-2 308 663 oder DE-A-3 119 349 aufgeführten Verbindungen geeignet. Weiterhin sind Barbitursaure und deren N-Substitutionsprodukte zu nennen. Als N-Substituenten kommen dabei insbesondere C₁-C₄-Alkyl oder Phenyl in Betracht.

Als Indolkupplungskomponenten sind beispielsweise 2-Methylindol, 2-Phenylindol, 2-Phenylindol-5-sulfonsäure, 1-Methyl-2-phenylindol, 1-(2'-Hydroxyethyl)-, 1-(2'-Carboxyethyl)-, 1-(2'-Carbamoylethyl)-2-methylindol oder -2-phenylindol zu nennen.

Als Pyridonkupplungskomponenten sind beispielsweise 1-Ethyl-2-hydroxy-4-methyl-5-carbamoylpyrid-6-on, 1-(2'-Hydroxyethyl)2-hydroxy-4-methyl-5-carbamoylpyrid-6-on, 1-Phenyl-2-hydroxy-4-methyl-5-carbamoylpyrid-6-on, 1-Ethyl-2-hydroxy-4-methyl-5-cyano-pyrid-6-on, 1-Ethyl-2-hydroxy-4-methyl-5-hydroxysulfonylmethyl-pyrid-6-on, 1-Methyl-2-hydroxy-4-methyl-5-cyanopyrid-6-on, 1-Methyl-2-hydroxy-5-acetylpyrid-6-on, 1,4-Dimethyl-2-hydroxy-5-cyanopyrid-6-on, 1,4-Dimethyl-5-carbamoylpyrid-6-on, 2,6-Di-hydroxy-4-ethyl-5-cyanopyridin, 2-Hydroxy-4-ethyl-5-carbamoyl-pyrid-6-on, 1-Ethyl-2-hydroxy-4-methyl-5-hydroxysulfonylmethyl-pyrid-6-on, 1-Methyl-2-hydroxy-4-methyl-5-methylsulfonylpyrid-6-on oder 1-Carboxymethyl-2-hydroxy-4-ethyl-5-phenylsulfonyl-pyrid-6-on zu nennen.

Faserreaktivgruppenhaltige Kupplungskomponenten KH der Naphthalin-, Benzol-, Pyrazolon-, Aminopyrazol-, 2,6-Diaminopyridin-, Pyridon-, Hydroxypyrimidin-, Aminopyrimidin-, Indoloder N-Arylacetoacetamidreihe sind beispielsweise Verbindungen der Formeln VIIIa-k 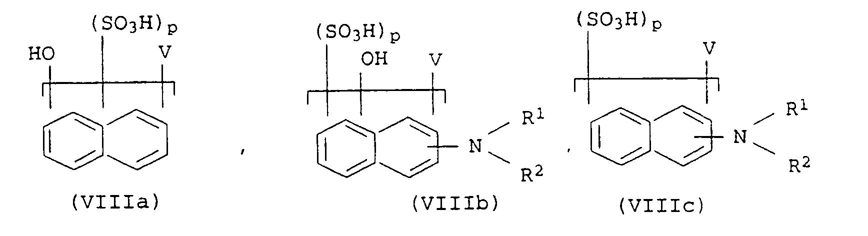 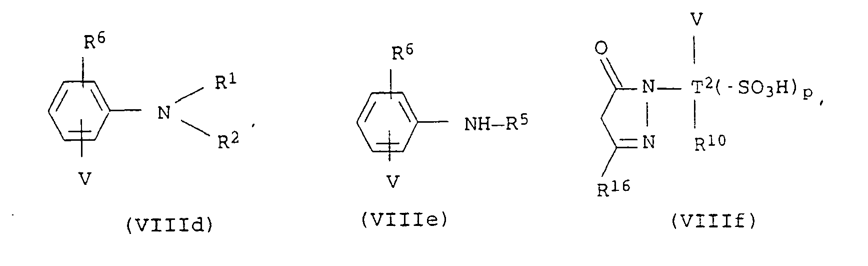 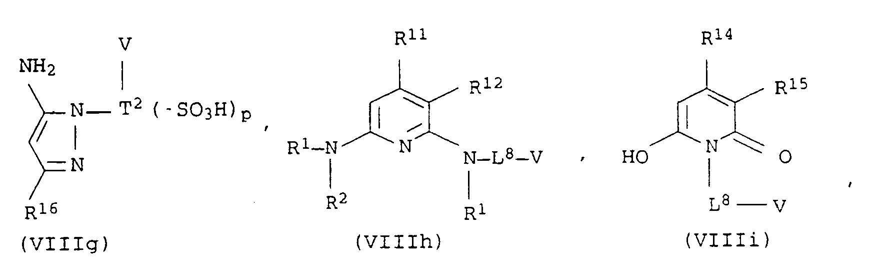 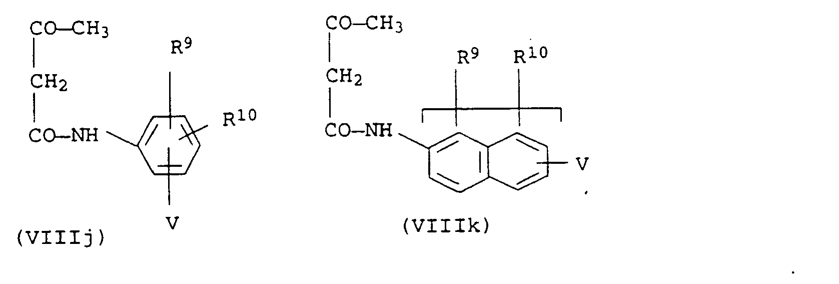 worin
T² für den Rest eines Benzol- oder Naphthalinrings,
R¹⁶ für Methyl, Carboxyl, C₁-C₄-Alkoxycarbonyl oder Phenyl und L⁸ für C₁-C₆-Alkylen stehen und R¹, R², R⁵, R⁶, R⁹, R¹⁰, R¹¹, R¹², R¹⁴, R¹⁵, p und V jeweils die obengenannte Bedeutung besitzen.

Faserreaktive Reste V tragende Pyrazolonkupplungskomponenten leiten sich beispielsweise von folgenden Pyrazolonen ab: 1-(3'-oder 4'-Aminophenyl)-, 1-(2'-Hydroxysulfonyl-5'-aminophenyl)-oder 1-(2'-Methoxy-5'-aminophenyl)- 3-carboxylpyrazol-5-on, 1-(3'- oder 4'-Aminophenyl)- oder 1-(6'-Amino- 4',8'-dihydroxy-sulfonylnaphth-2'-yl)-3-carboxylpyrazol-5-on.

Besonders bevorzugt ist die Verwendung von Farbstoffen der Formel IX

E-N=N-K¹ (IX),

in der E die obengenannte Bedeutung besitzt und K¹ für den Rest einer Kupplungskomponente der Benzol-, Naphthalin-, Pyrazol- oder Pyridinreihe steht, der gegebenenfalls weitere faserreaktive Gruppen aufweist, insbesondere die Gruppe E, die Gruppe der Formel SO₂-Y, worin Y die obengenannte Bedeutung besitzt, oder solche der Halogentriazinreihe, zum Farben von Haaren.

Insbesondere wird die Verwendung von Reaktivfarbstoffen mit aminosubstituierten Naphthalinen als Kupplungskomponenten bevorzugt, von denen sich vor allem die in 1-Position gekuppelten 2-Aminonaphthalinsulfonsauren auszeichnen. Die Farbstoffe der allgemeinen Formel X 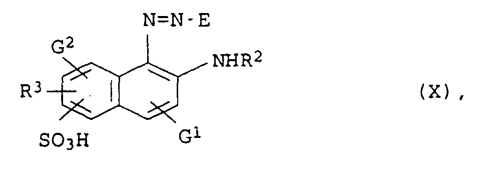 in der
- G¹: Wasserstoff oder Hydroxysulfonyl und
- G²: Wasserstoff oder Hydroxy bedeuten und

E, R² und R³ die obengenannte Bedeutung haben, sind daher vor allem bevorzugt.

Aus der Gruppe der Reaktivfarbstoffe der Formel X sind diejenigen hervorzuheben, in denen R² und/oder R³ für Wasserstoff, C₁-C₄-Alkyl, das durch Hydroxysulfonyl oder Carboxyl substituiert ist, insbesondere für Wasserstoff, Hydroxysulfonylmethyl oder Carboxymethyl, steht.

Aus der Gruppe der Farbstoffe der allgemeinen Formel X sind solche bevorzugt, in der R² und/oder R³ für Wasserstoff, Hydroxysulfonylmethyl oder Carboxymethyl, G¹ für Wasserstoff oder Hydroxysulfonyl, G² für Wasserstoff oder Hydroxy und im Rest E L³ für eine direkte Bindung und A¹ für Hydroxysulfonyl steht.

Weiterhin besonders bevorzugt ist die Verwendung von Farbstoffen der Formeln XIa und XIb 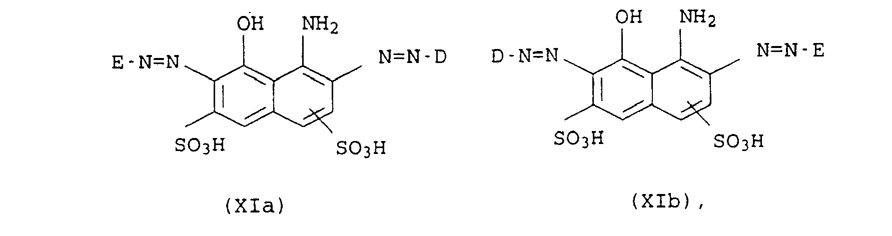 in denen der Rest E die obengenannte Bedeutung hat und D für den Rest einer Diazokomponente der Anilin- oder Naphthalinreihe steht, der gegebenenfalls weitere faserreaktive Gruppen, insbesondere die Gruppe E, die Gruppe SO₂Y oder solche der Halogentriazinreihe aufweist.

Weiterhin werden Reaktivfarbstoffe der Formel XII 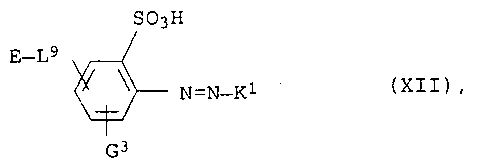 in der E und K¹ jeweils die obengenannte Bedeutung besitzen,
- L⁹: einen Rest der Formel O₂S-NZ¹, OC-NZ¹, Z¹N-SO₂, Z¹N-CO, Z¹N-CO-NZ², NZ¹ oder 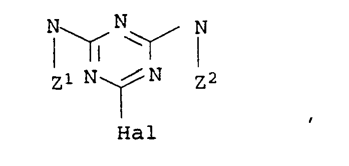 worin Z¹, Z² und Hal jeweils die obengenannte Bedeutung besitzen und
- G³: Wasserstoff, C₁-C₄-Alkyl, C₁-C₄-Alkoxy, Chlor oder Hydroxy-sulfonyl bedeuten, bevorzugt verwendet.

Weiterhin sind zur Haarfarbung Reaktivfarbstoffe der Formel XIII 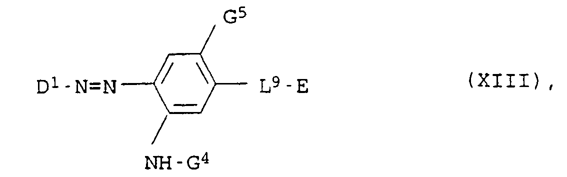 in der D¹, E und L⁹ jeweils die obengenannte Bedeutung besitzen,
- G⁴: für C₁-C₄-Alkanoyl, Carbamoyl, Mono- oder Di-(C₁-C₄)-alkyl-carbamoyl, Phenylcarbamoyl oder Cyclohexylcarbamoyl und
- G⁵: für Wasserstoff, C₁-C₄-Alkyl, C₁-C₄-Alkoxy, Hydroxysulfonyl oder Chlor stehen, besonders bevorzugt.

Weiterhin werden Reaktivfarbstoffe der Formeln XIVa und XIVb 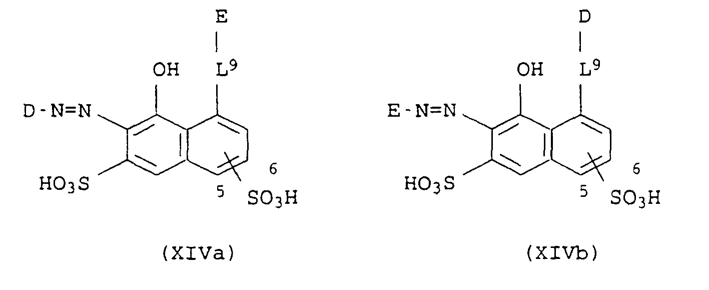 in denen D, E und L⁹ jeweils die obengenannte Bedeutung besitzen und die Hydroxysulfonylgruppe in Ringposition 5 oder 6 steht, bevorzugt verwendet.

Ferner wird die Verwendung von Reaktivfarbstoffen der Formel XV 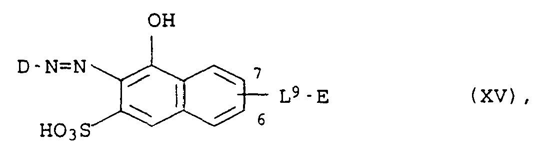 in der D, E und L⁹ jeweils die obengenannte Bedeutung besitzen und die Gruppe -L⁹-E in Ringposition 6 oder 7 steht, zur Haarfärbung bevorzugt.

Weiterhin sind Verbindungen der Formel XVI 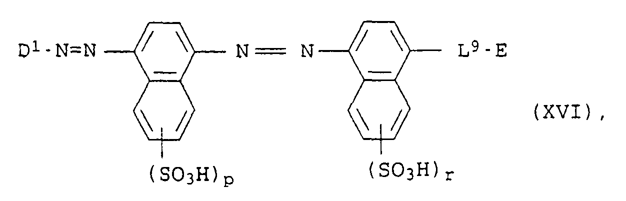 in der D¹, E und L⁹ jeweils die obengenannte Bedeutung besitzen und p und r unabhangig voneinander jeweils für 0, 1 oder 2 stehen, wertvoll.

Weiterhin sind Verbindungen der Formel XVII 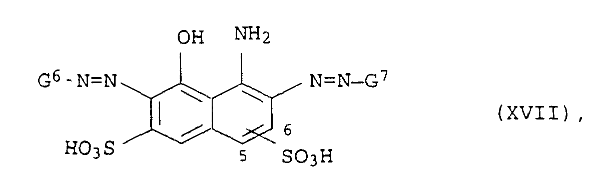 in der einer der beiden Reste G⁶ und G⁷ für den Rest D, wobei dieser die obengenannte Bedeutung besitzt,
und der andere für den Rest 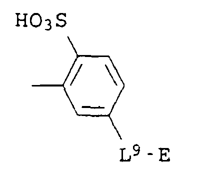 oder auch beide Reste G⁶ und G⁷ für den Rest 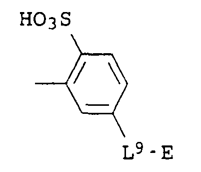 stehen, wobei L⁹ und E jeweils die obengenannte Bedeutung besitzen, besonders geeignet.

Anstelle der Azofarbstoffreste können die Farbstoffe der Formel I auch entsprechende Metallkomplexazofarbstoffreste enthalten. Als komplexierende Metalle kommen dabei insbesondere Kupfer, Kobalt Chrom, Nickel oder Eisen in Betracht, wobei Kupfer, Kobalt oder Chrom bevorzugt sind.

Dabei befinden sich die metallisierten Gruppen vorzugsweise jeweils in ortho-Stellung zur Azogruppe, z. B. in Form von o,o'-Dihydroxy-, o-Hydroxy-o'-carboxy-, o-Carboxy-o'-amino- oder o-Hydroxy-o'-amino-azogruppierungen.

W in Formel I stellt weiterhin z.B. den Rest eines metallisierten Formazanfarbstoffs dar, wobei insbesondere Kupferformazane zu nennen sind. Kupferformazane sind an sich bekannt und beispielsweise in K. Venkataraman "The Chemistry of Synthetic Dyes", Vol. III, Academic Press, New York, London, 1970, beschrieben.

Besonders bevorzugt sind Kupfer-Formazanfarbstoffe der Formel XVIII 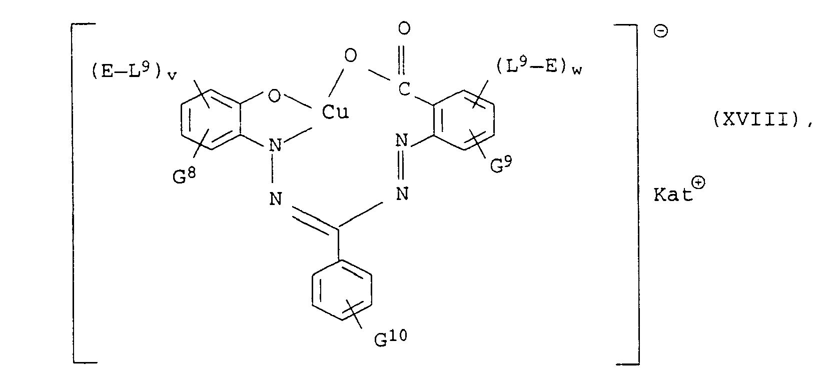 in der
- Kat^{⊕}: das Äquivalent eines Kations ist
- G⁸, G⁹ und G¹⁰: gleich oder verschieden sind und unabhangig voneinander jeweils Wasserstoff oder Hydroxysulfonyl
- v: 0 oder 1 und
- w: 0 oder 1 bedeuten und

E und L⁹ jeweils die obengenannte Bedeutung besitzen, mit der Maßgabe, daß v und w nicht gleichzeitig 0 bedeuten.

Kat^{⊕} in Formel XVIII stellt das Äquivalent eines Kations dar. Es ist entweder ein Proton oder leitet sich von Metall- oder Ammoniumionen ab. Metallionen sind insbesondere die Lithium-, Natrium- oder Kaliumionen. Unter Ammoniumionen im erfindungsgemäßen Sinne sind die bereits oben genannten unsubstituierten oder substituierten Ammoniumkationen zu verstehen.

Besonders als Kationen hervorzuheben sind Protonen oder Lithium-, Natrium- oder Kaliumionen, wobei die genannten Metallkationen auch bevorzugte Kationen sind, wenn die Reaktivfarbstoffe XVIII in Salzform vorliegen.

Eine Methode zur Herstellung der diesen Farbstoffen zugrundeliegenden Formazane ist beispielsweise in der EP-A-315 046 beschrieben.

W in Formel I stellt weiterhin z.B. den Rest eines Anthrachinonfarbstoffs dar. Anthrachinone sind an sich bekannt und beispielsweise in K. Venkataraman "The Chemistry of Synthetic Dyes", Vol. II, Academic Press, New York, 1952, beschrieben.

Besonders bevorzugt zum Färben von menschlichen Haar sind Anthrachinonfarbstoffe der Formel XIX 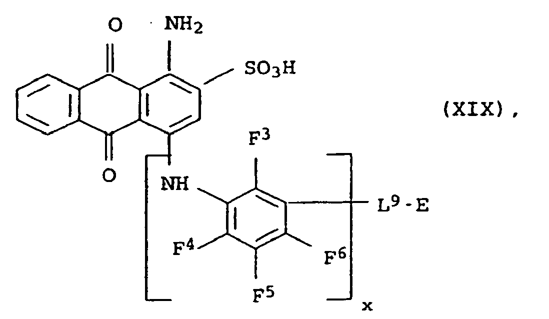 worin E und L⁹ jeweils die obengenannte Bedeutung besitzen,
- x: 0 oder 1
- F³ und F⁴: unabhängig voneinander jeweils Wasserstoff oder Methyl
und einer der beiden Reste F⁵ und F⁶ Wasserstoff oder Methyl und der andere Hydroxysulfonyl bedeuten.

W in Formel I stellt weiterhin z.B. den Rest eines Triphendioxazinfarbstoffs dar. Triphendioxazine sind an sich bekannt und beispielsweise in der EP-A-141 359 oder EP-A-311 969 beschrieben.

Beispielsweise sind Triphendioxazinfarbstoffe der Formel XX 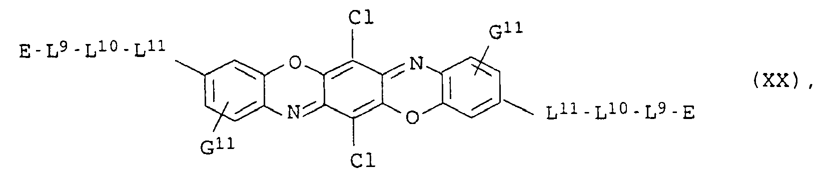 in der E und L⁹ jeweils die obengenannte Bedeutung besitzen und
- G¹¹: für Hydroxysulfonyl oder den Rest SO₂-C₂H₄-SO₃H,
- L¹⁰: für C₂-C₄-Alkylen oder Phenylen und
- L¹¹: für Sauerstoff, Imino oder C₁-C₄-Alkylimino stehen, geeignet.

W in Formel I stellt weiterhin z.B. den Rest eines metallisierten Phthalocyaninfarbstoffs dar. Phthalocyanine sind an sich bekannt und beispielsweise in F.H. Moser, D.L. Thomas "The Phthalocyanines", Vol. II, CRC Press, Boca Raton, Florida 1983, beschrieben.

Besonders bevorzugt sind Phthalocyaninfarbstoffe der Formel XXI 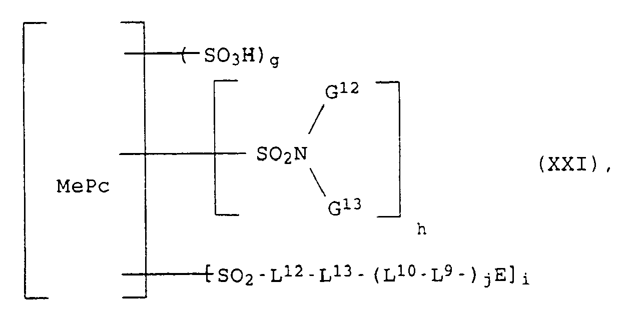 in der
- Pc: den Phthalocyaninrest,
- G¹² und G¹³: unabhängig voneinander jeweils Wasserstoff oder C₁-C₄-Alkyl,
- L¹²: Imino oder C₁-C₄-Alkylimino,
- L¹³: eine direkte Bindung oder C₁-C₄-Alkylen,
- Me: Kupfer oder Nickel,
- g: 0, 1 oder 2,
- h: 0, 1 oder 2
- i: 1 oder 2 und
- j: 0, 1, 2 oder 3 bedeuten
und E, L⁹ und L¹⁰ jeweils die obengenannte Bedeutung besitzen.

Weiterhin werden Reaktivfarbstoffe der Formel XXII 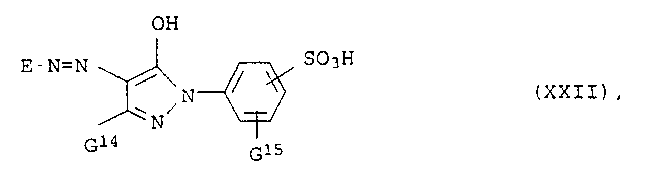 in der
G¹⁴ Methyl oder Carboxyl
G¹⁵ Wasserstoff oder Hydroxysulfonyl
und E die obengenannte Bedeutung besitzt, bevorzugt.

Ebenfalls bevorzugt sind Reaktivfarbstoffe der Formel XXIII 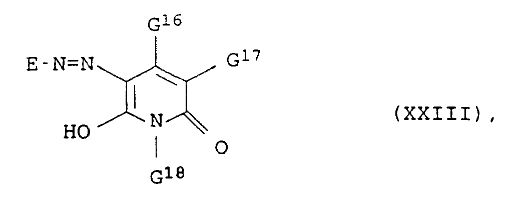 in der
G¹⁶ Wasserstoff oder Methyl
G¹⁷ Wasserstoff, Cyano, Carbamoyl oder Hydroxysulfonylmethyl
G¹⁸ Methyl oder Ethyl
und E die obengenannte Bedeutung besitzt.

Da in der Haarfarbung in der Regel keine Reinfarbstoffe verwendet werden, ist ausdrucklich auch die Verwendung von Mischungen von Farbstoffen der Formel I mit umfaßt.

Weiterhin kann man den Reaktivfarbstoffen der Formel I Direktfarbstoffe wie Azofarbstoffe, Anthachinonfarbstoffe oder Nitrofarbstoffe der Benzolreihe zufugen, um die erzeugten Farbtone abzustufen oder zu verstärken.

Die Herstellung der Reaktivfarbstoffe der Formel I kann nach an sich bekannten Methoden erfolgen.

Wenn der Rest W über eine Diazobrucke mit dem Ankerrest E verbunden ist, gelangt man zu den erfindungsgemaßen Farbstoffen, indem man z.B. die faserreaktive Verbindung der Formel XXIVa

H₂N-E (XXIVa),

in der E die obengenannte Bedeutung besitzt, auf an sich bekannte Weise diazotiert und mit einer Kupplungskomponente der Formel XXV

w¹-H (XXV)

kuppelt, in der W¹ den Rest einer Kupplungskomponente, eines Monoazofarbstoffs oder zusätzlich, wenn b=0, eines Disazofarbstoffs ist, die gegebenenfalls weitere reaktive Gruppen aufweisen, bedeutet.

Ist der Rest W hingegen über eines der unter Fall 2 aufgeführten Brückenglieder L⁹ mit dem Ankerrest E verbunden, so lassen sich die erfindungsgemaßen Farbstoffe beispielsweise derart herstellen, daß man einen geeigneten Farbstoff der Formel XXVI

W²-G¹⁹ (XXVI),

in der W² den Rest eines Chromophors, der gegebenenfalls weitere reaktive Gruppen aufweist und der sich von einem gegebenenfalls metallisierten Mono- oder Disazofarbstoff, einem Triphendioxazin, einem Anthrachinon, einem metallisierten Formazan oder einem metallisierten Phthalocyanin ableitet und G¹⁹ einen Aminorest der Formel NHZ¹ oder einen Saurehalogenidrest der Formel COHal oder SO₂Hal, worin Z¹ und Hal jeweils die obengenannte Bedeutung besitzen, bedeuten, mit einer faserreaktiven Verbindung der Formel XXIVb

G²⁰-E (XXIVb),

in der E die obengenannte Bedeutung besitzt und G²⁰ einen der Reste G¹⁹ bedeutet umsetzt, mit der Maßgabe, daß jeweils ein Amin mit einem Säurehalogenid reagiert.

Wenn L⁹ (Fall 2) eine Harnstoffbrücke oder einen Triazinrest darstellt, fügt man die bei diesen Stoffklassen üblichen Syntheseschritte ein.

Es ist auch möglich, von solchen Vorprodukten der Verbindungen der Formel XXVI auszugehen, die ein Teil des späteren Chromophors sind, und sie zunächst mit der faserreaktiven Verbindung XXIVb umzusetzen und anschließend den Chromophorrest W² aufzubauen.

Wenn b in Formel I den Wert 1 bedeutet, so kann man zu den verbrückten Chromophoren z.B. auf die Weise gelangen, daß man entweder die fertigen Einzelchromophore verbrückt oder aber zunächst geeignete Zwischenprodukte verbrückt und danach die jeweiligen chromophoren Systeme aufbaut.

Weiterhin sind beispielsweise in der US-A-4066 638, EP-A-107 614, EP-A-107617, EP-A-559 617, EP-A-581 729, EP-A-693 536, DE-A-3441272, DE-A-3441273, DE-A-4437265, EP-A-637615, EP-A-318968, JP-A-7118830, JP-A-7128904, NL-A-7410432, EP-A-637615, DE-A-19600765, DE-A-4434989, DE-A-19523245 DE-A-2 154 942 sowie der älteren deutschen Patentanmeldung DE-A-19611667 Farbstoffe beschrieben, die unter die allgemeine Formel I fallen.

Die Verwendung der in diesen Dokumenten als Beispiele aufgeführten Farbstoffe zur Haarfärbung ist ausdrücklich mit umfaßt.

Weiterhin betrifft die vorliegende Anmeldung kosmetische Zubereitungen, die neben den für die Haarfärbung üblichen Hilfsmittel die obigen Farbstoffe enthalten.

Die Farbstoffe werden dabei in gelöster Form in einem wäßrigen kosmetisch verträglichen Milieu angewendet. Im wäßrigen kosmetisch verträglichen Träger variiert der pH-Wert zwischen 5 und 9 und beträgt vorzugsweise 6 - 8. Er wird mit Hilfe von milden anorganischen oder organischen Basen, Salzen schwachen Säuren oder Puffern auf den gewunschten Wert eingestellt. Beispielhaft sind Ammoniak, Ammonium-, Kalium- oder Natriumcarbonat, Natriumhydroxid wie Mono-, Di- oder Triethanolamin, Dinatriumhydrogenphosphat, Natriumcitrat oder Natriumborat, zu nennen.

Die Farbstoffe sind in den Haarfarbemittel in Anteilen von 0,01 bis 10 Gew.-% bezogen auf das Gesamtgewicht der Zubereitung enthalten.

Übliche Hilfsmittel in kosmetischen Zubereitungen für die Haarfarbung sind anionische, kationische, nichtionische oder amphotere oberflächenaktive Verbindungen oder Gemische hiervon. Als oberflächenaktive Verbindung sind Seifen, Alkylbenzolsulfonate, Alkylnaphthalinsulfonate, Sulfate, Ethersulfate und Sulfonate von Fettalkoholen, quarternäre Ammoniumsalze, wie Trimethylcetylammoniumbromid, Cetylpyridiniumbromid, Quaternium 1 bis X (INCI), Cocoyltrimethylammoniummethylsulfat (INCI), Hydroxyethyl Cetyldimomium Phosphate (INCI), Cetyltrimethylammoniumchlorid, gegebenenfalls oxyethylenierte Fettsäureethanolamide, polyoxyethylenierte Säuren, Alkohole und Amine, polyglycerinierte Alkohole, polyoxyethylenierte oder polyglycerinierte Alkylphenole sowie polyoxyethylenierte Alkylsulfate zu nennen. Die oberflächenaktiven Verbindungen sind in den erfindungsgemaßen Mitteln zu 0,5 bis 40 Gew.-%, bezogen auf das Gesamtgewicht der Zubereitung enthalten.

Weitere übliche Hilfsmittel sind organische Lösungsmittel als Solubilisierungsmittel z.B. C₁-C₄-Alkohol wie Ethanol und Isopropanol, Glykole, Glykolether wie Ethylenglykol, Propylenglykol, 2-Methoxyethanol, 2-Ethoxyethanol oder 2-Butoxyethanol, sowie Glycerin. Die Lösungsmittel sind in der Regel von 0 - 40 Gew.-% bezogen auf das Gesamtgewicht der Zubereitung enthalten.

Um die Handhabung der Zubereitung zu vereinfachen werden üblicherweise Verdickungsmittel den erfindungsgemaßen Zubereitungen als Hilfsmittel zugesetzt. Übliche Verdickungsmittel sind Cellulosederivate wie Methyl-, Hydroxymethyl-, Hydroxyethyl-, Hydroxypropyl- oder Carboxymethylcellulose, Natriumalginat, Gummi arabikum, Xanthangummi, Traganth, Acrylsäurepolymere, Polyvinylpyrrolidon, Vinylacetat-Crotonsäure-Copolymere, Vinylacetat-Vinylpyrrolidon-Copolymere, Butylvinylether-Maleinsäureanhydrid-Copolymere, Methylvinylether-Maleinsäureanhydrid-Copolymere, oder ein anorganisches Verdickungsmittel wie Bentonit. Diese Verdickungsmittel werden in der Regel bis zu 5 Gew.-% bezogen auf das Gesamtgewicht der Zubereitung eingesetzt.

Haarkosmetische Zubereitungen, die in Form von Gelen angewandt werden sollen, enthalten noch gelbildende Substanzen wie beispielsweise Carbomere (INCI). Zur Einstellung bestimmter pflegender Eigenschaften können die Zubereitungen zusätzlich kationische Polymere und Siliconverbindungen enthalten. Geeignete kationische Polymere sind z.B. Polyquaternium 1 bis x nach INCI, Copolymere aus Vinylpyrrolidon/Vinylimidazoliumsalzen (Luviquat® FC, Luviquat HM, Hersteller: BASF), Copolymere aus Vinylpyrroli-don/Dimethylaminoethylmethacrylat, quaterniert mit Diethylsulfat (Luviquat PQ 11); kationische Cellulosederivate (Polyquaternium-4 und 10), Acrylamidocopolymere (Polyquaternium-7) und kationische Guar-Gum-Derivate, z.B. Guar Hydroxypropyltriminium Chloride (INCI). Geeignete Siliconverbindungen sind z.B. Polyalkylsiloxane, Polyarylsiloxane, Polyarylalkylsiloxane, Polyethersiloxane oder Siliconharze.

Weitere übliche Hilfsmittel sind milde Antioxidantien, die keine Reaktionen mit den Farbstoffen eingehen, Pentrationsmittel, Sesquestriermittel, Puffer, Parfumol, Lichtschutzmittel (UV-A und UV-B-Filter), Konservierungsmittel, Haarreinigungspraparate und Wirkstoffe wie Pantheonol, Bisabolol und Vitamine z.B. des Typs A, C und E.

Die erfindungsgemaßen kosmetischen Zubereitungen können in flussiger Form in der Regel verdickt, als Creme, Paste, als Gel oder in irgendeiner anderen geeigneten Form zum Färben von Haaren angewendet werden.

In einer bevorzugten Anwendung wird die Zubereitung auf das Haar aufgetragen, 5 bis 50 Minuten, vorzugsweise 10 bis 30 Minuten einwirken gelassen und anschließend das Haar gespült und gegebenenfalls mit einem herkömmlichen Schampoo gewaschen.

Durch eine warme Zubereitung oder Wärmeeinwirkung von außen, kann die Färbung beschleunigt oder bei gleicher Einwirkungsdauer verstärkt werden. Bevorzugt werden Temperaturen im Bereich von 20 bis 40°C gewählt.

Die Reaktivfarbstoffen der Formel I ergeben gleichmaßige Färbungen und decken auch weiße Haare gut ab. Die Färbungen zeigen gegenüber äußeren Einflusse eine gute Lichtechtheit, Waschechtheit, Witterungsechtheit und Abriebechtheit.

Weiterhin ermöglichen Reaktivfarbstoffe aufgrund ihres anderen Färbeprinzip den Verzicht auf H₂O₂ als Oxidationsmittel im Färbeverfahren. Besonders vorteilhaft ist dabei die Tatsache, daß der Farbton durch den Farbstoff vorgegeben ist und nicht erst im Haar entwickelt wird. Dies vereinfacht die Herstellung von Farbstoffmischungen und Nuancierungen.

Von ihrer Verwendung als Textilfarbstoffe ist ferner ihre physiologische Unbedenklichkeit bekannt.

Die nachfolgenden Beispiele sollen die Erfindung erläutern.

### Beispiel 1:

1,25 g des Reaktivfarbstoffs der Formel 1 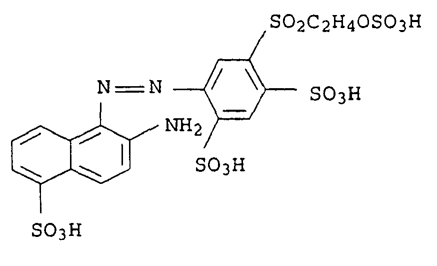 wurden in 25 ml Wasser gelost und mit Natriumhydrogenphosphat ein pH-Wert von 7 eingestellt. In die auf 36°C erwarmt Losung wurde eine gebleichte Strahne menschlichen Haares (2 g) getaucht und die Lösung 20 Minuten bei dieser Temperatur einwirken gelassen. Danach wurde das Haar mit Wasser gespult und an der Luft getrocknet. Man erhielt eine kraftige scharlachrote Haarfarbung (λ max = 508 nm, Remission).

Unter denselben Bedingungen wurden Farbungen mit den in den folgenden Beispielen aufgeführten Farbstoffen erhalten, wobei E¹ und E² für die Reste der Formel 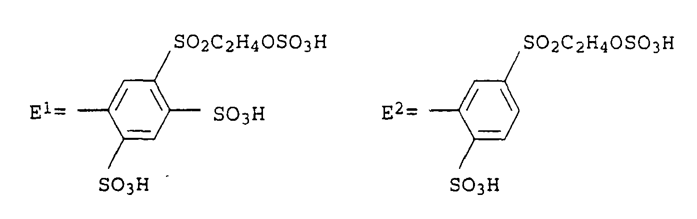 stehen.

### Beispiel 2

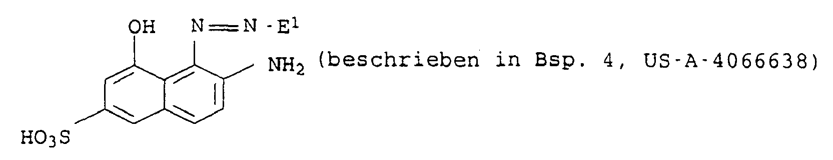

blaustichiges Rot (λ max = 534, Remission)

### Beispiel 3

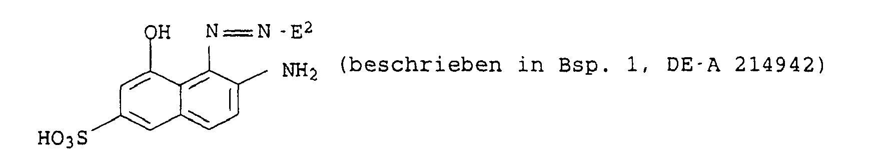

blaustichiges Rot (λ max = 539, Remission)

### Beispiel 4

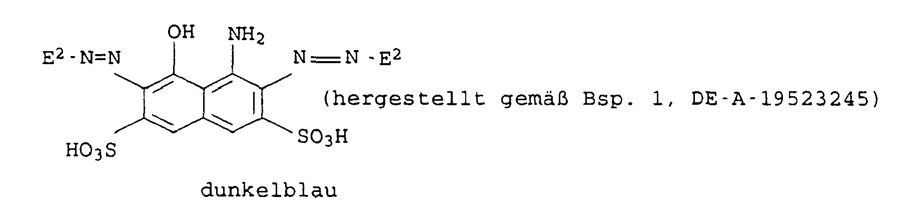

### Beispiel 5

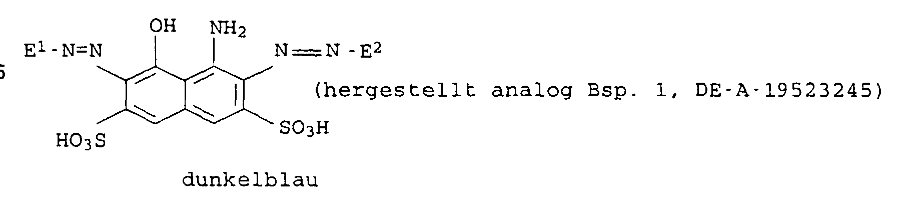

### Beispiel 6

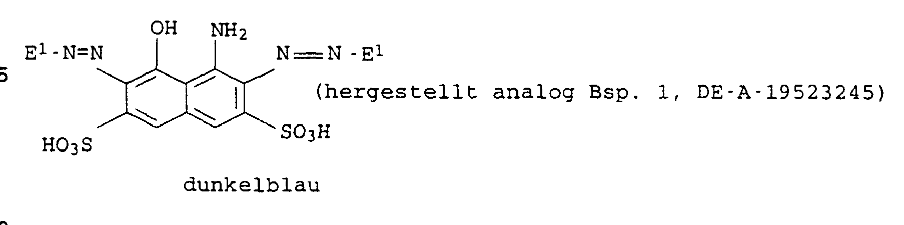

### Beispiel 7

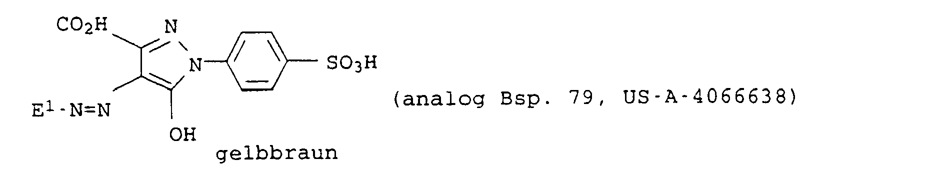

### Herstellvorschrift zu Beispiel 1:

131 g 1-Amino-3-(β-sulfatoethylsulfonyl)benzol-4,6-disulfonsaure wurden in 700 ml Eiswasser und 60 ml Salzsaure (30 Gew.-%ig) angeruhrt und bei 0 - 5 °C portionsweise mit 60 ml einer 23 Gew.-%igen Natriumnitrit-Lösung versetzt. Nach beendeter Diazotierung wurde der schwache Nitrituberschuß mit etwas Amidosulfonsaure zerstort. Zu dieser Reaktionsmischung gab man bei 0 - 5 °C 60 g 2-Aminonaphthalin-5-sulfonsaure in 800 ml Eiswasser, hielt mit Natriumacetat einen pH von 2 - 3 ein und ließ langsam auf 20 °C erwärmen. Nachdem keine Diazoverbindung mehr nachweisbar war, wurde der pH-Wert durch Zugabe von Natriumhydrogencarbonat auf 5 gestellt und klärfiltriert. Der Farbstoff wurde durch Zugabe von 500 g Natriumchlorid gefallt, abgesaugt und im Vacuum bei 40 °C getrocknet. Man erhielt ein sich leicht in Wasser lösendes rotliches Pulver. Ein ähnlicher Farbstoff ist in der US-A-406638 in Beispiel 3 beschrieben.

Die Farbstoffe in den Beispielen 2, 3 und 7 wurden in analoger Weise hergestellt.

### Formulierungsbeispiele:

### A) Haarfarbecreme

| Phase I: | |
|---|---|
| 1,5 g | Ceteareth-6 (and) Stearyl Alcohol (INCI) |
| 1.5 g | Ceteareth-25 |
| 6,0 g | Cetearyl Octanoate |
| 3,0 g | Cetearyl Alcohol |

| Phase II: | |
|---|---|
| 2 g | Reaktivfarbstoff der Formel 1 |
| 2 g | Propylenglycol |
| 84 g | Wasser dest. |
| q.s. | Citronensäure/Triethanolamin zur Einstellung des pH-Wertes auf pH 7 |
| q.s. | Konservierungsmittel |

| Phase III: | |
|---|---|
| q.s. | Parfümöl |

Die Einsatzstoffe wurden bei 60°C gelöst und anschließend die Phase I in die Phase II gegeben. Nach Abkühlen auf 30°C wurde Phase III zugegeben.

Eine gebleichte Strahne menschlichen Haares (2 g) wurde mit 0,5 g der Färbecreme behandelt und 20 min einwirken lassen. Anschließend wurde mit Wasser ausgespült. Man erhielt eine wie in Beispiel 1 beschriebene Haarfarbung.

### B) Haarfarbelotion

| | |
|---|---|
| 5g | Reaktivfarbstoff der Formel 1 |
| 1,2 g | Natrosol® 250 HR (Hersteller: Aqualon), (Hydroxyethyl-cellulose nach INCI) |
| 1g | Propylenglycol |
| ad 100 g | Wasser dest. |
| q.s. | Konservierungsmittel |

Eine gebleichte Strahne menschlichen Haares (2 g) wurde mit 0,5 g der Farbelotion behandelt und 20 min einwirken lassen. Anschließend wurde mit Wasser ausgespult. Man erhielt eine wie in Beispiel 1 beschriebene Haarfarbung.

### C) Haarfarbe - Mousse

| | |
|---|---|
| 2 g | Reaktivfarbstoff der Formel 1 |
| 3 g | Luviskol® VA 64 (Hersteller: BASF), (PVP/VA Copolymer nach INCI) |
| 0,45 g | Ceteareth-25 (INCI) |
| 0,10 g | Dimethicone (INCI) |
| 10 g | Propan/Butan |
| ad 100 g | Wasser dest. |
| q.s. | Konservierungsmittel |

Eine gebleichte Haarstrahne menschlichen Haares (2 g) wurde mit 0,5 g des Haarfarbe-Mousses behandelt, 15 min einwirken lassen und anschließend mit Wasser gespült. Neben einer intensiven Färbung laßt sich das Haar gleichzeitig sehr leicht durchkammen und wirkt gepflegt. Man erhielt eine wie in Beispiel 1 beschriebene Haarfarbung.

### D) Haarfarbeshampoo

| | |
|---|---|
| 5 g | Reaktivfarbstoff der Formel 1 |
| 40 g | Natriumlaurylethersulfat (Texapon® N 28, Hersteller: Henkel) (Sodium Laurylethersulfate nach INCI) |
| 10 g | Tego® Betain L 7 (Hersteller: Goldschmidt) (Cocamidopropyl Betaine nach INCI) |
| 2 g | Gluatin WQ (Weizenkeimprotein) |
| ad 100 g | Wasser dest. |
| q.s. | Konservierungsmittel |
| q.s. | Kochsalz als Verdicker |

Die Verwendung eines Haarfarbeshampoos ermöglicht die gleichzeitige Reinigung und Färbung des Haares. Eine gebleichte Haarsträhne menschlichen Haares (2 g) wurde mit 0,5 g des Haarfarbeshampoos behandelt und nach 1 min ausgespult bis kein Schäumen mehr auftrat. Man erhielt eine wie in Beispiel 1 beschriebene Haarfarbung.

### E) Farbepaste

| | |
|---|---|
| 2 g | Reaktivfarbstoff der Formel 1 |
| 7 g | Titandioxid |
| 15 g | Aerosil® (Hersteller: Degussa) |
| 10 g | Lutrol® F 127 (Polyethylenglycol, Hersteller: BASF) |
| ad 100 g | Wasser dest. |
| q.s. | Konservierungsmittel |

Eine gebleichte Haarstrahne menschlichen Haares (2 g) wurde mit 0,5 g der Haarfärbe-Paste behandelt, 15 min einwirken lassen und anschließend mit Wasser gespult. Man erhielt eine wie in Beispiel 1 beschriebene Haarfarbung.

### F) Haarfarbeschampoo

| | |
|---|---|
| 10 g | Reaktivfarbstoff der Formel 1 |
| 2,20 g | Xanthangummi (Keltrol® T, Hersteller: Kelco) |
| 20,0 g | Natriumlaurylethersulfat |
| 2,50 g | Oleindiethanolamid |
| 0,10 g | Trilon® B (Hersteller: BASF) |
| ad 100 g | Wasser dest. |
| q.s. | Konservierungsmittel |

25 g der Färbepaste wurden mit 25 ml Wasser dest. angerührt, eine gebleichte Strähne menschlichen Haares (2 g) mit diesem Färbeshampoo behandelt und 20 min einwirken gelassen. Anschließend wurde mit Wasser ausgespult. Man erhielt eine wie in Beispiel 1 beschriebene Haarfärbung.

## Patentansprüche

1. Verwendung von Reaktivfarbstoffen der Formel I 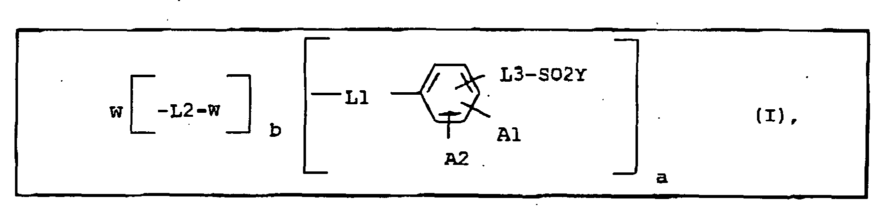 in der
a 1 oder 2,
b 0 oder 1,
Y Vinyl oder einen Rest der Formel C₂H₄Q, worin Q für eine unter alkalischen Reaktionsbedingungen abspaltbare Gruppe steht,
L³ eine direkte Bindung oder ein Brückenglied der Formel CO-NH-M¹, worin M¹ für C₂-C₆-Alkylen steht, daß durch 1 oder 2 nicht benachbarte Sauerstoffatome, Imino- oder C₁-C₄-Alkyliminogruppen unterbrochen sein kann,
A¹ Hydroxysulfonyl oder einen Rest der Formel SO₂Y
A² Wasserstoff, Hydroxysulfonyl, Methoxy, Chlor, Brom oder Carboxyl
W
im Fall 1) den Rest einer Kupplungskomponente, eines Monoazofarbstoffs oder zusätzlich, wenn b = 0, eines Disazofarbstoffs, die weitere faserreaktive Gruppen tragen können, oder
im Fall 2) den Rest eines Chromophors, der gegebenenfalls weitere faserreaktive Gruppen aufweist und der sich von einem gegebenenfalls metallisierten Mono-oder Disazofarbstoff, einem Triphendioxazin, einem Anthrachinon, einem metallisierten Formazan oder
L¹
im Fall 1) eine Azobrücke oder
im Fall 2) ein Brückenglied der Formel O₂S-NZ¹, OC-NZ¹, Z¹N-SO₂, Z¹N-CO, Z¹N-CO-NZ², NZ¹ oder 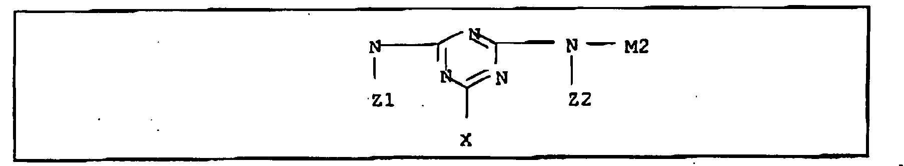 worin M² für eine direkte Bindung oder Methylen, Z¹ und Z² unabhängig voneinander jeweils für Wasserstoff, C₁-C₆-Alkyl oder Phenyl und X für Fluor, Chlor oder Brom, Amino, C₁-C₆-Alkylamino, das gegebenenfalls durch 1 oder 2 nicht benachbarte Sauerstoffatome, Imino- oder C₁-C₄-Alkyliminogruppe unterbrochen und gegebenenfalls mit Hydroxy substituiert ist, N-Pyrrolidinyl, N-Piperidinyl, N-Morpholinyl, N-Piperazinyl oder N-(C₁-C₄)-Alkylpiperazinyl oder NZ¹ oder NZ² auch für Piperazin-1,4-diyl stehen,
L² einen Rest der Formel 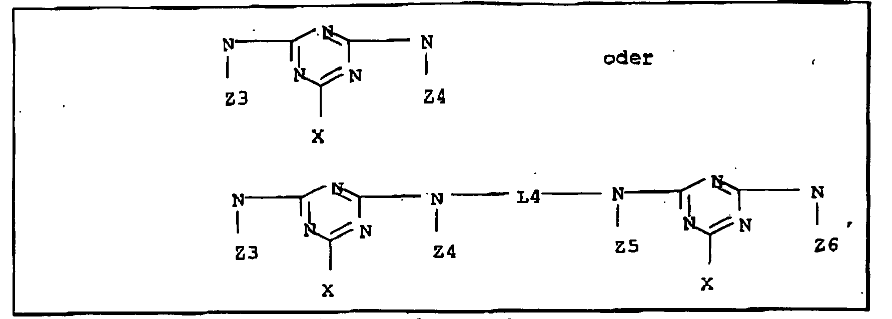 worin Z³, Z⁴, Z⁵ und Z⁶ unabhängig voneinander jeweils für Wasserstoff, C₁-C₆-Alkyl oder Phenyl und L⁴ für C₂-C₈-Alkylen oder gegebenenfalls durch C₁-C₄-Alkyl, C₁-C₄-Alkoxy oder Hydroxysulfonyl substituiertes Phenylen stehen und X jeweils die obengenannte Bedeutung besitzt, bedeuten
zum Färben von menschlichem Haar.

2. verwendung von Reaktivfarbstoffen nach Anspruch 1, **dadurch gekennzeichnet, daß** L³ eine direkte Bindung bedeutet.

3. Verwendung von Reaktivfarbstoffen nach Anspruch 1 oder 2, **dadurch gekennzeichnet, daß** A¹ Hydroxysulfonyl bedeutet.

4. Verwendung von Reaktivfarbstoffen nach den Ansprüchen 1 bis 3, **dadurch gekennzeichnet, daß** A² Wasserstoff oder Hydroxysulfonyl bedeutet.

5. Verwendung von Reaktivfarbstoffen nach den Ansprüchen 1 bis 4, **dadurch gekennzeichnet, daß** L¹ eine Azobrücke ist.

6. Verwendung von Reaktivfarbstoffen nach den Ansprüchen 1 bis 4, **dadurch gekennzeichnet, daß** L¹ im Fall 2) ein Brückenglied der Formel 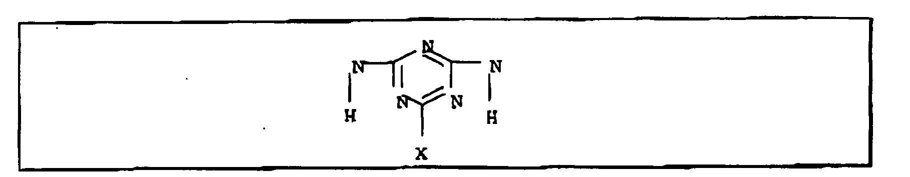 bedeutet.

7. Verwendung von Reaktivfarbstoffen nach den Ansprüchen 1 bis 6, **dadurch gekennzeichnet, daß** Y vinyl oder einen Rest der Formel C₂H₄OSO₃H bedeutet.

8. Verfahren zum Färben von menschlichem Haar, **dadurch gekennzeichnet, daß** als Farbstoffe Reaktivfarbstoffe gemäß den Ansprüchen 1 bis 7 verwendet werden.

## Claims

1. The use of reactive dyes of the formula I 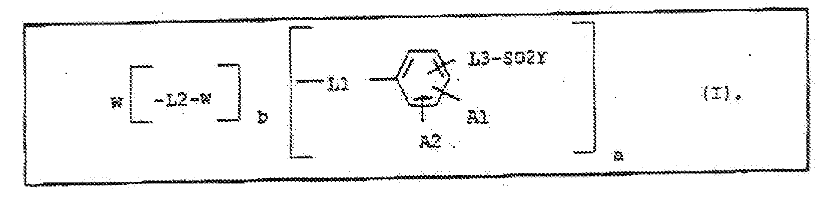 where
a is 1 or 2,
b is C or 1,
Y is vinyl or a radical of the formula C₂H₄Q, where Q is an alkali-detachable group,
L is a direct bond or a bridge member of the formula CO-NH-M¹, where M¹ is C₂-C₆-alkylene with or without interruption by 1 or 2 unadjacent oxygen atoms, imino or C₁-C₄-alkylimino groups.
A¹ is hydroxysulfonyl or a radical of the formula SC₂Y,
A² is hydrogen, hydroxysulfonyl, methoxy, chlorine, bromine or carboxyl,
W is either in case 1) the radical of a coupling component, of a monoazo dye or additionally, when b = 0, of a disazo dye, which may each bear further fiber-reactive groups, or
in case 2) the radical of a chromophore which optionally has further fiber-reactive groups and is derived from an optionally metallized mono- or disazo dye, from a triphendioxazine from an anthraquinone, from a metallized formazan or from a metallized phthalocyanine,
L¹ is either in case 1) an azo bridge or
in case 2) a bridge member of the formula O₂S-NZ¹, OC-NZ¹, Z¹N-SO₂, Z¹N-CO, Z¹N-CO-NZ², NZ¹ or 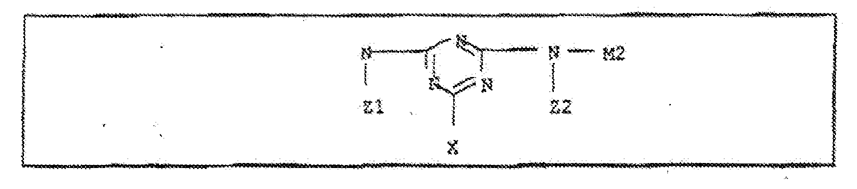 where M² is a direct bond or methylene, Z¹ and Z² are each independently of the other hydrogen, C₁-C₆-alkyl or phenyl and X is fluorine, chlorine or bromine, amino, C₁-C₆-alkylamino with or without interruption by 1 or 2 unadjacent oxygen atoms, imino or C₁-C₄-alkylinino groups and with or without hydroxyl substitution, N-pyrrolidinyl, N-piperidinyl, N-morpholinyl, N-piperazinyl or N-(C₁-C₄)-alkylpiperazinyl, or NZ¹ or NZ² each also represent 1,4-piperazinediyl,
L² is a radical of the formula 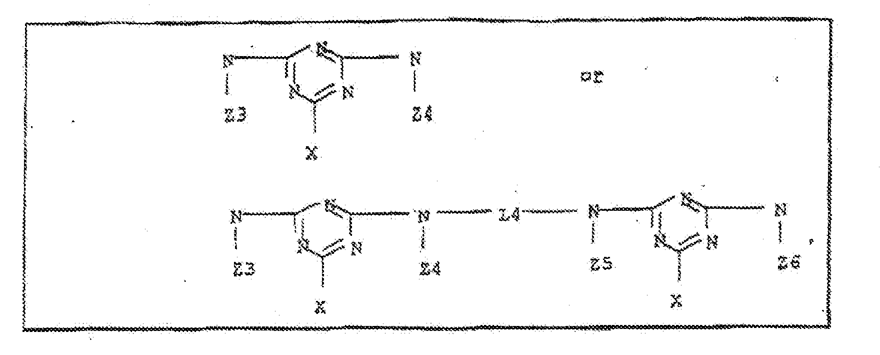 where Z³, Z⁴, Z⁵ and Z⁶ are each independently of the others hydrogen, C₁-C₆-alkyl or phenyl, L⁴ is C₂-C₈-alkylene or unsubstituted or C₁-C₄-alkyl-, C₁-C₄-alkoxy- or hydroxysulfonyl-substituted phenylene, and X is in each case as defined above,
for dyeing human hair.

2. The use according to claim 1, wherein L² is a direct bond.

3. The use according to claim 1 or 2, wherein A¹ is hydroxysulfonyl.

4. The use according to any of claims 1 to 3, wherein A² is hydrogen or hydroxysulfonyl.

5. The use according to any of claims 1 to 4, wherein L¹ is an azo bridge.

6. The use according to any of claims 1 to 4, wherein L¹ is a bridge member of the formula 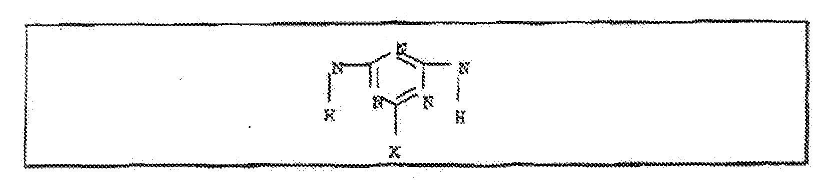 in case 2).

7. The use according to any of claims 1 to 6, wherein Y is vinyl or a radical of the formula C₂H₄OSO₃H.

8. A method of dyeing human hair, which comprises using reactive dyes according to any of claims 1 to 7.

## Revendications

1. Utilisation de colorants réactifs de formule I 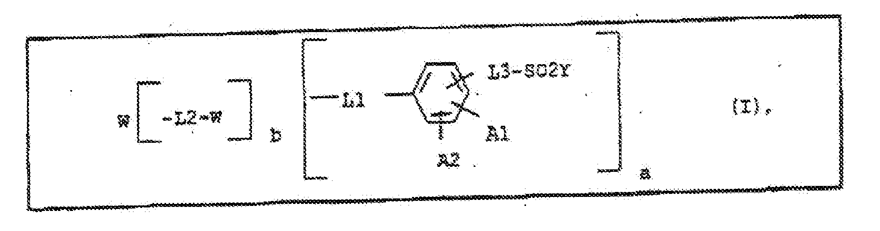 dans laquelle
a est 1 ou 2,
b est 0 ou 1,
Y représente le groupe vinyle ou un radical de formule C₂H₄Q, dans lequel Q représente un groupe séparable dans des conditions réactíonnelles alcalínes,
L³ représente une liaison directe ou un chaînon pontant de formule CO-NH-M¹, dans lequel M¹ représente un groupe alkylène en C₂-C₆, qui peut être interrompu par 1 ou 2 atomes d'oxygène ou groupes imino ou alkyl(C₁-C₄)imino non contigus,
A¹ représente le groupe hydroxysulfonyle ou un radical de formule SO₂Y,
A² représente un atome d'hydrogène, de chlore ou de brome ou un groupe hydroxysulfonyle, méthoxy ou carbozy,
W représente
dans le cas 1) le reste d'un composant de copulation, d'un colorant monoazoïque ou en outre, lorsque b = 0, d'un colorant disazoique qui peut porter d'autres groupes réactifs avec les fíbres, ou
dans le cas 2) le reste d'un chromophore qui comporte éventuellement d'autres groupes réactifs avec les fibres et qui dérive d'un colorant mono- ou disazoïque éventuellement métallé, d'une triphènedioxaxine d'une anthraquinone, d'un formazan métallé ou d'une phtalocyanìne métallée,
L¹ représente
dans le cas 1) un pont azo ou
dans le cas 2) un chaînon pontant de formule O₂S-NZ¹, OC-NZ¹, Z¹N-SO₂, Z¹N-CO, Z¹H-CO-NZ², NZ¹ ou 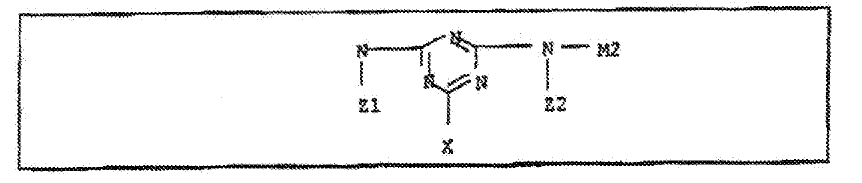 où M² représente une liaison directe ou un groupe méthylène, Z¹ et Z² représentent chacun, indépendamment l'un de l'autre, un atome d'hydrogène, un groupe alkyle en C₁-C₆ ou phènyle et X représente un atome de fluor, chlore ou brome, un groupe amino, alkyl (C₁-C₆) amino qui est éventuellement substitué par 1 ou 2 atomes d'oxygène ou groupes imino ou alkyl(C₁-C₄)imino, non contigus et éventuellement par hydroxy, un groupe N-pyrrolidinyle, N-pipéridinyle, N-morpholinyle, N-pipérazinyle ou N-alkyl(C₁-C₄)-pipérazinyle ou NZ¹ ou NZ² représent également un groupe pipérazine-1,4-diyle,
L² représente un radical de formule 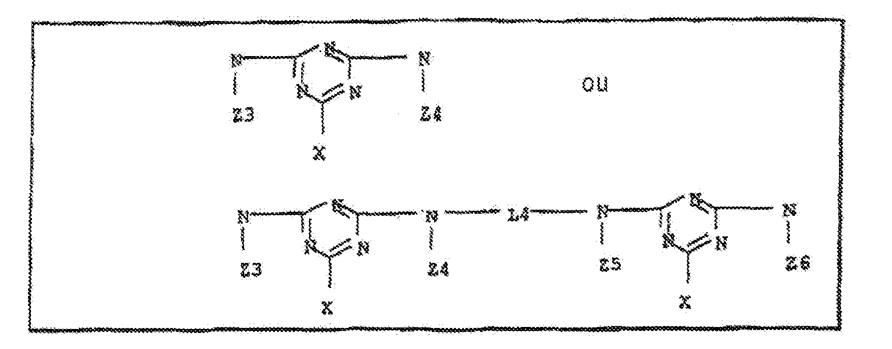 dans laquelle Z³, Z⁴, Z⁵ et Z⁶ représentent chacun, indépendamment les uns des autres, un atome d'hydrogène, un groupe alkyle en C₁-C₆ ou phényle, et L⁴ représente un groupe alkylène en C₂-C₈ ou phénylène éventuellement substitué par alkyle en C₁-C₄, alcoxy en C₁-C₄
ou hydroxysulfonyle, et X a chaque fois la signification donnée plus haut,
pour la teinture des cheveux humains.

2. Utilisation de colorants réactifs selon la revendication 1, **caractérisée en ce que** L³ représente une liaison directe.

3. Utilisation de colorants rèactifs selon la revendication 1 ou 2, **caractérisée en ce que** A¹ représente un groupe hydroxysulfonyle.

4. Utilisation de colorants réactifs selon les revendications 1 à 3, **caractérisée en ce que** A² représente un atome d'hydrogène ou le groupe hydroxysulfonyle.

5. Utilisation de colorants réactifs selon les revendications 1 à 4, **caractérisée en ce que** L¹ est un pont azo.

6. Utilisation de colorants réactifs selon les revendications 1 à 4, **caractérisée en ce que** L¹ dans le cas 2) représente un chaînon pontant de formule

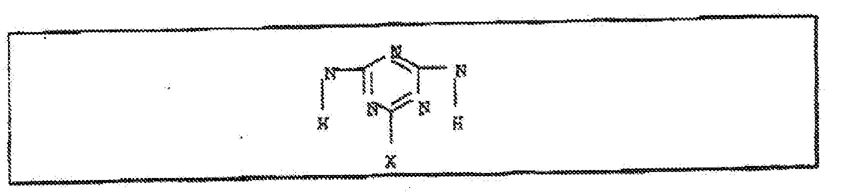

7. Utilisation de colorants réactifs selon les revendications 1 à 6, **caractérisée en ce que** Y représente le groupe vinyle ou un radical de formule C₂H₄OSO₃H.

8. Procédé pour la teinture des chevaux humains, **caractérisé en ce qu'**on utilise en tant que colorants des colorants réactifs selon les revendications 1 à 7.
